# EUROPEAN PATENT SPECIFICATION

(11) **EP 1 754 713 B1**
(45) Date of publication and mention of the grant of the patent: **25.02.2009**
(21) Application number: 06013626.4
(22) Date of filing: 29.04.2004
(51) Int. Cl.: C07K 7/04, A61K 38/04, C07K 14/705, C12N 15/10

(54) **Apo2L (Trail) receptor binding peptides and uses thereof**
Peptide welche an Apo2L (Trail) Rezeptoren binden und Verwendungen davon
Peptides se liant aux récepteurs Apo2L (Trail) et utilisations desdits peptides

(30) Priority: 09.05.2003 US 469436 P
(43) Date of publication of application: 21.02.2007
(62) Divisional of application: 04760861.7
(73) Proprietor: Genentech, Inc., South San Francisco CA 94080-4990 (US)
(72) Inventor: Li, Bing, Foster City CA 94044 (US); Sidhu, Sachdev S., San Francisco CA 94110 (US)
(74) Representative: Kiddle, Simon John

(56) References cited:
- CHUNTHARAPAI A ET AL: "Isotype-dependent inhibition of tumor growth in vivo by monoclonal antibodies to death receptor 4." JOURNAL OF IMMUNOLOGY (BALTIMORE, MD. : 1950) 15 APR 2001, vol. 166, no. 8, 15 April 2001 (2001-04-15), pages 4891-4898, XP002300581 ISSN: 0022-1767
- GRIFFITH T S ET AL: "FUNCTIONAL ANALYSIS OF TRAIL RECEPTORS USING MONOCLONAL ANTIBODIES" JOURNAL OF IMMUNOLOGY, THE WILLIAMS AND WILKINS CO. BALTIMORE, US, vol. 162, 1999, pages 2597-2605, XP002944456 ISSN: 0022-1767
- ICHIKAWA K ET AL: "Tumoricidal activity of a novel anti-human DR5 monoclonal antibody without hepatocyte cytotoxicity" NATURE MEDICINE, NATURE AMERICA, NEW YORK, US, vol. 7, no. 8, August 2001 (2001-08), pages 954-960, XP002956334 ISSN: 1078-8956
- ASHKENAZI AVI: "Targeting death and decoy receptors of the tumour-necrosis factor superfamily." NATURE REVIEWS. CANCER. JUN 2002, vol. 2, no. 6, June 2002 (2002-06), pages 420-430, XP002300582 ISSN: 1474-175X

## Description

### FIELD OF THE INVENTION

The present invention relates to peptides which bind to receptors for Apo-2L for use in methods of treating cancer or immune-related diseases.

### BACKGROUND OF THE INVENTION

Various molecules, such as tumor necrosis factor-alpha ("TNF-alpha"), tumor necrosis factor-beta ("TNF-beta" or "lymphotoxin-alpha.), lymphotoxin-beta ("LT-beta"), CD30 ligand, CD27 ligand, CD40 ligand, OX-40 ligand, 4-1BB ligand, Apo-1 ligand (also referred to as Fas ligand or CD95 ligand), Apo-2 ligand (also referred to as Apo2L or TRAIL), Apo-3 ligand (also referred to as TWEAK), APRIL, OPG ligand (also referred to as RANK ligand, ODF, or TRANCE), and TALL-1 (also referred to as BlyS, BAFF or THANK) have been identified as members of the tumor necrosis factor ("TNF") family of cytokines (See, e.g., Gruss and Dower, Blood, 85:3378-3404 (1995); Schmid et al., Proc. Natl. Acad. Sci., 83:1881 (1986) ; Dealtry et al., Eur. J. Immunol., 17:689 (1987); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997; Marsters et al., Curr. Biol., 8:525-528 (1998); Chicheportiche et al., Biol. Chem., 272:32401-32410 (1997); Hahne et al., J. Exp. Med., 188:1185-1190 (1998); WO98/28426 published July 2, 1998; WO98/46751 published October 22, 1998; WO98/18921 published May 7, 1998; Moore et al., Science, 285:260-263 (1999); Shu et al., J. Leukocyte Biol., 65:680 (1999); Schneider et al., J. Exp. Med., 189:1747-1756 (1999); Mukhopadhyay et al., J. Biol. Chem., 274:15978-15981 (1999)). Among these molecules, TNF-alpha, TNF-beta, CD30 ligand, 4-1BB ligand, Apo-1 ligand, Apo-2 ligand (Apo2L/TRAIL) and Apo-3 ligand (TWEAK) have been reported to be involved in apoptotic cell death.

Apo2L/TRAIL was identified several years ago as a member of the TNF family of cytokines. (see, e.g., Wiley et al., Immunity, 3:673-682 (1995); Pitti et al., J. Biol. Chem., 271:12697-12690 (1996)) The full-length human Apo2L/TRAIL polypeptide is a 281 amino acid long, Type II transmembrane protein. Some cells can produce a natural soluble form of the polypeptide, through enzymatic cleavage of the polypeptide's extracellular region (Mariani et al., J. Cell. Biol., 137:221-229 (1997)). Crystallographic studies of soluble forms of Apo2L/TRAIL reveal a homotrimeric structure similar to the structures of TNF and other related proteins (Hymowitz et al., Molec. Cell, 4:563-571 (1999); Hymowitz et al., Biochemistry, 39:633-644 (2000)). Apo2L/TRAIL, unlike other TNF family members however, was found to have a unique structural feature in that three cysteine residues (at position 230 of each subunit in the homotrimer) together coordinate a zinc atom, and that the zinc binding is important for trimer stability and biological activity. (Hymowitz et al., supra; Bodmer et al., J. Biol. Chem., 275:20632-20637 (2000))

It has been reported in the literature that Apo2L/TRAIL may play a role in immune system modulation, including autoimmune diseases such as rheumatoid arthritis, and in the treatment of HIV (see, e.g., Thomas et al., J. Immunol., 161:2195-2200 (1998); Johnsen et al., Cytokine, 11:664-672 (1999); Griffith et al., J. Exp. Med., 189:1343-1353 (1999); Song et al., J. Exp. Med., 191:1095-1103 (2000); Jeremias et al., Eur. J. Immunol., 28:143-152 (1998); Katsikis et al., J. Exp. Med., 186:1365-1372 (1997); Miura et al., J. Exp. Med., 193:651-660 (2001)).

Soluble forms of Apo2L/TRAIL have also been reported to induce apoptosis in a variety of cancer cells *in vitro*, including colon, lung, breast, prostate, bladder, kidney, ovarian and brain tumors, as well as melanoma, leukemia, and multiple myeloma (see, e.g., Wiley et al., supra; Pitti et al., supra; Rieger et al., FEBS Letters, 427:124-128 (1998); Ashkenazi et al., J. Clin. Invest., 104:155-162 (1999); Walczak et al., Nature Med., 5:157-163 (1999); Keane et al., Cancer Research, 59:734-741 (1999); Mizutani et al., Clin. Cancer Res., 5:2605-2612 (1999); Gazitt, Leukemia, 13:1817-1824 (1999); Yu et al., Cancer Res., 60:2384-2389 (2000); Chinnaiyan et al., Proc. Natl. Acad. Sci., 97:1754-1759 (2000)). *In vivo* studies in murine tumor models further suggest that Apo2L/TRAIL, alone or in combination with chemotherapy or radiation therapy, can exert substantial anti-tumor effects (see, e.g., Ashkenazi et al., supra; Walzcak et al., supra; Gliniak et al., Cancer Res., 59:6153-6158 (1999); Chinnaiyan et al., supra; Roth et al., Biochem. Biophys. Res. Comm., 265:1999 (1999)). In contrast to many types of cancer cells, most normal human cell types appear to be resistant to apoptosis induction by certain recombinant forms of Apo2L/TRAIL (Ashkenazi et al., supra; Walzcak et al., supra). Jo et al. has reported that a polyhistidine-tagged soluble form of Apo2L/TRAIL induced apoptosis *in vitro* in normal isolated human, but not non-human, hepatocytes (Jo et al., Nature Med., 6:564-567 (2000); see also, Nagata, Nature Med., 6:502-503 (2000)). It is believed that certain recombinant Apo2L/TRAIL preparations may vary in terms of biochemical properties and biological activities on diseased versus normal cells, depending, for example, on the presence or absence of a tag molecule, zinc content, and % trimer content (See, Lawrence et al., Nature Med., Letter to the Editor, 7:383-385 (2001); Qin et al., Nature Med., Letter to the Editor, 7:385-386 (2001)).

Induction of various cellular responses mediated by such TNF family cytokines is believed to be initiated by their binding to specific cell receptors. Previously, two distinct TNF receptors of approximately 55-kDa (TNFR1) and 75-kDa (TNFR2) were identified (Hohman et al., J. Biol. Chem., 264:14927-14934 (1989); Brockhaus et al., Proc. Natl. Acad. Sci., 87:3127-3131 (1990); EP 417,563, published March 20, 1991; Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)). Those TNFRs were found to share the typical structure of cell surface receptors including extracellular, transmembrane and intracellular regions. The extracellular portions of both receptors were found naturally also as soluble TNF-binding proteins (Nophar, Y. et al., EMBO J., 9:3269 (1990); and Kohno, T. et al., Proc. Natl. Acad. Sci. U.S.A., 87:8331 (1990); Hale et al., J. Cell. Biochem. Supplement 15F, 1991, p. 113 (P424)).

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. (Schall et al., supra; Loetscher et al., supra; Smith et al., supra; Nophar et al., supra; Kohno et al., supra; Banner et al., Cell, 73:431-435 (1993)). A similar repetitive pattern of CRDs exists in several other cell-surface proteins, including the p75 nerve growth factor receptor (NGFR) (Johnson et al., Cell, 47:545 (1986); Radeke et al., Nature, 325:593 (1987)), the B cell antigen CD40 (Stamenkovic et al., EMBO J., 8:1403 (1989)), the T cell antigen OX40 (Mallet et al., EMBO J., 9:1063 (1990)) and the Fas antigen (Yonehara et al., supra and Itoh et al., Cell, 66:233-243 (1991)). CRDs are also found in the soluble TNFR (sTNFR)-like T2 proteins of the Shope and myxoma poxviruses (Upton et al., Virology, 160:20-29 (1987); Smith et al., Biochem. Biophys. Res. Commun., 176:335 (1991); Upton et al., Virology, 184:370 (1991)). Optimal alignment of these sequences indicates that the positions of the cysteine residues are well conserved. These receptors are sometimes collectively referred to as members of the TNF/NGF receptor superfamily.

The TNF family ligands identified to date, with the exception of lymphotoxin-beta, are typically type II transmembrane proteins, whose C-terminus is extracellular. In contrast, most receptors in the TNF receptor (TNFR) family identified to date are typically type I transmembrane proteins. In both the TNF ligand and receptor families, however, homology .. identified between family members has been found mainly in the extracellular domain ("ECD"). Several of the TNF family cytokines, including TNF-alpha, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Pan et al. have disclosed another TNF receptor family member referred to as "DR4" (Pan et al., Science, 276:111-113 (1997); see also WO98/32856 published July 30, 1998). The DR4 was reported to contain a cytoplasmic death domain capable of engaging the cell suicide apparatus. Pan et al. disclose that DR4 is believed to be a receptor for the ligand known as Apo2L/TRAIL.

In Sheridan et al., Science, 277:818-821 (1997) and Pan et al., Science, 277:815-818 (1997), another molecule believed to be a receptor for Apo2L/TRAIL is described (see also, WO98/51793 published November 19, 1998; WO98/41629 published September 24, 1998). That molecule is referred to as DR5 (it has also been alternatively referred to as Apo-2; TRAIL-R, TR6, Tango-63, hAPO8, TRICK2 or KILLER (Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/0265 published January 21, 1999; WO99/09165 published February 25, 1999; WO99/11791 published March 11, 1999). Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

A further group of recently identified receptors are referred to as "decoy receptors," which are believed to function as inhibitors, rather than transducers of signaling. This group includes DCR1 (also referred to as TRID, LIT or TRAIL-R3) (Pan et al., Science, 276:111-113 (1997); Sheridan et al., Science, 277:818-821 (1997); McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)) and DCR2 (also called TRUNDD or TRAIL-R4) (Marsters et al., Curr. Biol., 7:1003-1006 (1997); Pan et al., FEBS Letters, 424:41-45 (1998); Degli-Esposti et al., Immunity, 7:813-820 (1997)), both cell surface molecules, as well as OPG (Simonet et al., supra; Emery et al., infra) and DCR3 (Pitti et al., Nature, 396:699-703 (1998)), both of which are secreted, soluble proteins. Apo2L/TRAIL has been reported to bind those receptors referred to as DcR1, DcR2 and OPG.

Apo2L/TRAIL is believed to act through the cell surface "death receptors" DR4 and DR5 to activate caspases, or enzymes that carry out the cell death program. Upon ligand binding, both DR4 and DR5 can trigger apoptosis independently by recruiting and activating the apoptosis initiator, caspase-8, through the death-domain-containing adaptor molecule referred to as FADD/Mort1 (Kischkel et al., Immunity, 12:611-620 (2000); Sprick et al., Immunity, 12:599-609 (2000); Bodmer et al., Nature Cell Biol., 2:241-243 (2000)). In contrast to DR4 and DR5, the DcR1 and DcR2 receptors do not signal apoptosis.

For a review of the TNF family of cytokines and their receptors, see Ashkenazi and Dixit, Science, 281:1305-1308 (1998); Ashkenazi and Dixit, Curr. Opin. Cell Biol., 11:255-260 (2000); Golstein, Curr. Biol., 7:750-753 (1997) ; Gruss and Dower, supra; Nagata, Cell, 88:355-365 (1997); Locksley et al., Cell, 104:487-501 (2001).

Moreover, monoclonal antibodies to human DR4 CTRAIL-R1) and DR5 (TRAIL-R2) receptors that inhibit the binding of TRAIL to said receptors are known in the art (Chuntharapai et al., Journal of Immunology, 166(8): 4891-4898 (2001); Griffith et al., Journal of Immunology, 162: 2597-2605 (1999); Ichikawa et al., Nature Medecine, 7(8): 954-960).

### SUMMARY OF THE INVENTION

The present invention provides peptides for use in methods of treating cancer or immune-related diseases that bind receptors for Apo-2 ligand (also referred to as Apo-2L or TRAIL). Optionally, the peptide is in monomer, dimer, trimer, tetramer, or higher oligomeric forms. Optionally the peptide is a chimeric molecule comprising the Apo-2L receptor binding peptide fused to a heterologous peptide sequence facilitating the formation of oligomeric complexes (e.g. a leucine zipper domain). In one embodiment, the peptide inhibits the interaction of Apo-2L with at least one of its receptors, such as the DR5 receptor. Optionally, the compound is an agonist of at least one Apo-2L associated biological activity, for example, the induction of apoptosis via the DR5 receptor.

In certain embodiments, the peptides are an Apo-2L receptor binding peptide having about 10 to about 25 amino acid residues and comprising an amino acid sequence selected from the group consisting of SEQ ID NO: 14 through SEQ ID NO: 104 (Table VIII).

As shown, for example, in Table VIII, Apo-2L receptor binding peptides preferably exhibit certain features or characteristics. For example, in preferably exhibit certain features or characteristics. For example, in certain embodiments of the invention, the Apo-2L receptor binding peptides have a cysteine residue at position 1, 2, 3, 4, 5, 6, or 7 numbered from its N-terminus. In preferred embodiments, the Apo-2L receptor binding peptide includes the following motif: cysteine-X₁-X₂-X₃-cysteine, wherein X₁ is an amino acid residue selected from the group consisting of methionine and leucine, X₂ is an amino acid residue selected from the group consisting of threonine and alanine, and X₃ is an amino acid residue selected from the group consisting of serine, methionine, leucine and glutamic acid.

The Apo-2L receptor binding peptides may be modified using one of the wide variety of methods known in the art. In preferred embodiments of the invention, an Apo-2L receptor binding peptide of the invention is linked to a heterologous molecule or polypeptide sequence. Optionally, the heterologous polypeptide sequence is a leucine zipper domain. Optionally the heterologous sequence comprises the amino acid sequence glycine-glycine-methionine. Optionally, the Apo-2L receptor binding peptide may be conjugated or linked to one or more linker molecules or polyol groups.

In certain embodiments of the invention, the Apo-2L receptor binding peptide inhibits the interaction between Apo-2L and DR4. In some embodiments of the invention, the Apo-2L receptor binding peptide inhibits the interaction between Apo-2L and DR5. Optionally, the Apo-2L receptor binding peptide induces apoptosis in one or more mammalian cells.

The peptides may be formulated in a composition comprising at least one of the peptides described above in a carrier. Preferably, this composition is sterile.

In particularly desirable embodiments, the resulting compositions are pharmaceutically acceptable formulations.

Other embodiments of the invention are methods of modulating the biological activity of Apo-2L and/or an Apo-2L receptor in mammalian cells. A preferred embodiment of the invention is a method of inducing apoptosis in mammalian cells, comprising exposing mammalian cells to an effective amount of an Apo-2L receptor binding peptide described herein. Another embodiment of the invention is a method of inhibiting an Apo-2L associated biological activity such as apoptosis in mammalian cells, comprising exposing mammalian cells to an effective amount of an Apo-2L receptor binding peptide described herein. The mammalian cells may be, e.g., cancer cells. In the present invention, the cancer or cancer or an immune related disorder is a breast, lung, or colon (or colorectal) cancer, arthritis or multiple sclerosis. The peptides described herein can be administered alone or together with another agent.

### BRIEF DESCRIPTION OF THE DRAWINGS

Figure 1 shows the nucleotide sequence of human Apo-2 ligand cDNA (SEQ ID NO:2) and its derived amino acid sequence (SEQ ID NO:1). The "N" at nucleotide position 447 (in SEQ ID NO:2) is used to indicate the nucleotide base may be a "T" or "G".
Figures 2A and 2B show the nucleotide sequence of a cDNA for full length human DR4 (SEQ ID NO:3) and its derived amino acid sequence (SEQ ID NO:4). The respective nucleotide and amino acid sequences for human DR4 are also reported in Pan et al., Science, 276:111 (1997).
Figure 3A shows the 411 amino acid sequence of human DR5 (SEQ ID NO:5) as published in WO 98/51793 on November 19, 1998. A transcriptional splice variant of human DR5 is known in the art. This DR5 splice variant encodes the 440 amino acid sequence of human DR5 shown in Figures 3B and 3C (SEQ ID NO:6) as published in WO 98/35986 on August 20, 1998.
Figures 4A-4D provide illustrations of the protocols used to modify Apo-2L receptor binding peptides.
Figure 5A is a schematic representation of the G4 (LB881) peptide linked to a heterologous leucine zipper polypeptide. The complete amino acid sequence of this polypeptide is GEVCLTSCSRLRGGSGGM KLKQIEDKLEEILSKLYHIENELARIKKLVGER (SEQ ID NO: 113). Figure 5B is a graphic representation showing the apoptosis-inducing activity of an oligomerized LB881 Apo2L binding peptide (SEQ ID NO: 113). The apoptosis-inducing activity of this molecule was assessed using SK-MES-1 lung carcinoma cells and an alamar blue assay (as described in Example 4 below).

### DETAILED DESCRIPTION OF THE INVENTION

### A. DEFINITIONS

"TNF family member" is used in a broad sense to refer to various polypeptides that share some similarity to tumor necrosis factor (TNF) with respect to structure or function. Certain structural and functional characteristics associated with the TNF family of polypeptides are known in the art and described, for example, in the above Background of the Invention. Such polypeptides include but are not limited to those polypeptides referred to in the art as TNF-alpha, TNF-beta, CD40 ligand, CD30 ligand, CD27 ligand, OX-40 ligand, 4-1BB ligand, Apo-1 ligand (also referred to as Fas ligand or CD95 ligand), Apo-2L/TRAIL (also referred to as TRAIL), Apo-3 ligand (also referred to as TWEAK), APRIL, OPG ligand (also referred to as RANK ligand, ODF, or TRANCE), and TALL-1 (also referred to as BlyS, BAFF or THANK) (See, e.g., Gruss and Dower, Blood 1995, 85:3378-3404; Pitti et al., J. Biol. Chem. 1996, 271:12687-12690; Wiley et al., Immunity 1995, 3:673-682; Browning et al., Cell 1993, 72:847-856; Armitage et al. Nature 1992, 357:80-82, PCT Publication Nos. WO 97/01633; and WO 97/25428; Marsters et al., Curr. Biol. 1998, 8:525-528; Chicheportiche et al., Biol. Chem. 1997, 272:32401-32410; Hahne et al., J. Exp. Med. 1998, 188:1185-1190; PCT Publication Nos. WO98/28426; WO98/46751; and WO98/18921; Moore et al., Science 1999, 285:260-263; Shu et al., J. Leukocyte Biol. 1999, 65:680; Schneider et al., J. Exp. Med. 1999, 189:1747-1756; Mukhopadhyay et al., J. Biol. Chem. 1999, 274:15978-15981).

"Apo-2L receptor binding peptide" is used in a broad sense to refer to peptides that bind to one or more Apo-2L receptors. The term "Apo-2L receptor binding peptide" exclude antibodies directed against the DR4 and/or DR5 receptors. Preferably, the peptide has about 10 to about 25 amino acid residues, or about 10 to about 20 residues, and more preferably about 12 to about 20 amino acid residues. Optionally, the Apo-2L receptor binding peptide consists of no more than 25 amino acid residues (e.g., 25 or less amino acid residues). Preferably, the peptide has a cysteine residue at position 1, 2, 3, 4, 5, 6 or 7 numbered from its N-terminus. Still more preferably, the peptide additionally has a valine, glutamic acid, or glycine residue N-terminal to the N-terminal cysteine residue. Even more preferably, the peptide is such that the residue N-terminal to the N-terminal cysteine residue is a valine residue. Still more preferably, the peptide has a motif comprising the following: cysteine-X₁-X₂-X₃-cysteine. Preferably, X₁ is either a methionine or a leucine residue. Preferably X₂ is a threonine or alanine residue. Preferably X₃ is a serine, methionine, leucine or glutamic acid residue. This peptide even more preferably has a leucine residue at position 2 numbered from its C-terminus. Optionally the Apo-2L receptor binding peptide is fused or linked to a heterologous sequence or molecule. Preferably the heterologous sequence allows or assists the peptide to form higher order or oligomeric complexes (e.g. a leucine zipper domain). Optionally, the Apo-2L receptor binding peptide binds to a first Apo-2L receptor (e.g. DR5) but not a second Apo-2L receptor (e.g. DR4, DcR1, DcR2). Alternatively, the Apo-2L receptor binding peptide binds to a first Apo-2L receptor (e.g. DR5) and further binds to one or more other Apo-2L receptors (e.g. DR4, DcR1, DcR2). Optionally the peptide is an antagonist which inhibits the interaction of Apo-2L with DR5. Alternatively, the peptide is an agonist of DR5 signalling activity. Optionally the peptide is an antagonist which inhibits the interaction of Apo-2L with DR4. Alternatively, the peptide is an agonist of DR4 signalling activity.

Optionally, the Apo-2L receptor binding peptides of the invention bind to an Apo-2L receptor (e.g., DR5 or DR4) at a concentration range of about 1 nM to about 1 mM (and optionally of about 100nM to about 1mM) as measured in a peptide binding assay (such as disclosed in the Examples below). Optionally, the Apo-2L receptor binding peptides of the invention exhibit a Ic 50 value of about 3 nm to about 150 nm (and preferably of about 6 nm to about 50 nm) as measured in a peptide binding assay (such as disclosed in the Examples below).

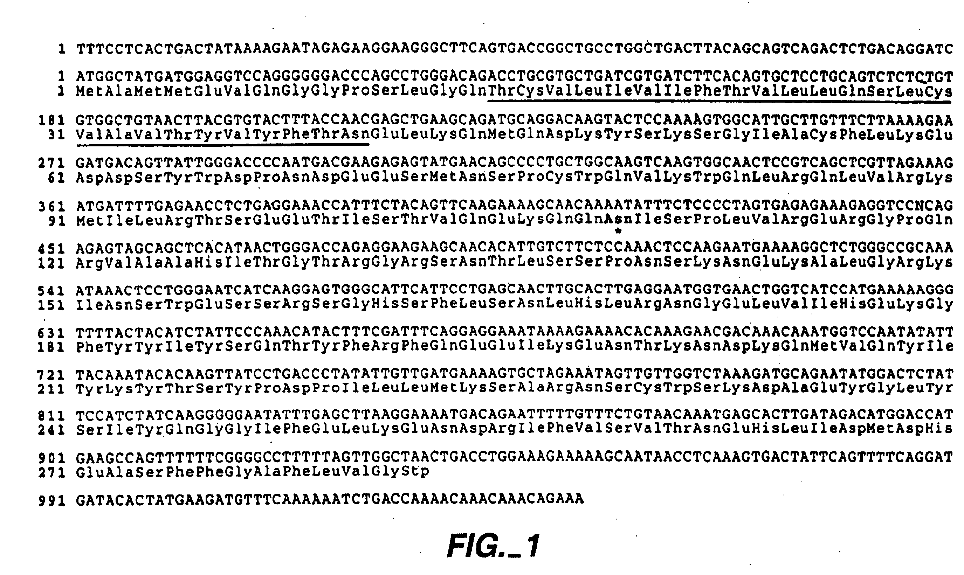

The terms "Apo2L/TRAIL", "Apo-2L", and "TRAIL" are used herein to refer to a polypeptide sequence which includes amino acid residues 114-281, inclusive, 95-281, inclusive, residues 92-281, inclusive, residues 91-281, inclusive, residues 41-281, inclusive, residues 15-281, inclusive, or residues 1-281, inclusive, of the amino acid sequence shown in Figure 1 (SEQ ID NO:1), as well as biologically active fragments, deletional, insertional, or substitutional variants of the above sequences. In one embodiment, the polypeptide sequence comprises residues 114-281 of Figure 1 (SEQ ID NO:1), and optionally, consists of residues 114-281 of Figure 1 (SEQ ID NO:1). Optionally, the polypeptide sequence comprises residues 92-281 or residues 91-281 of Figure 1 (SEQ ID NO:1). The Apo-2L polypeptides may be encoded by the native nucleotide sequence shown in Figure 1 (SEQ ID NO:2). Optionally, the codon which encodes residue Pro119 (Figure 1; SEQ ID NO: 2) may be "CCT" or "CCG". In other embodiments, the fragments or variants are biologically active and have at least about 80% amino acid sequence identity, more preferably at least about 90% sequence identity, and even more preferably, at least 95%, 96%, 97%, 98%, or 99% sequence identity with any one of the above recited Apo2L/TRAIL sequences. Optionally, the Apo2L/TRAIL polypeptide is encoded by a nucleotide sequence which hybridizes under stringent conditions with the encoding polynucleotide sequences provided in Figure 1 (SEQ ID NO:2). The definition encompasses substitutional variants of Apo2L/TRAIL in which at least one of its native amino acids are substituted by an alanine residue. Particular substitutional variants of the Apo2L/TRAIL include those in which at least one amino acid is substituted by an alanine residue. These substitutional variants include those identified, for example, as "D203A"; "D218A" and "D269A." This nomenclature is used to identify Apo2L/TRAIL variants wherein the aspartic acid residues at positions 203, 218, and/or 269 (using the numbering shown in Figure 1 (SEQ ID NO:1)) are substituted by alanine residues. Optionally, the Apo2L variants may comprise one or more of the alanine substitutions which are recited in Table I of published PCT application WO 01/00832. Substitutional variants include one or more of the residue substitutions identified in Table I of WO 01/00832 published January 4, 2001. The definition also encompasses a native sequence Apo2L/TRAIL isolated from an Apo2L/TRAIL source or prepared by recombinant or synthetic methods. The Apo2L/TRAIL of the invention includes the polypeptides referred to as Apo2L/TRAIL or TRAIL disclosed in PCT Publication Nos. WO97/0163 and WO97/25428. The terms "Apo2L/TRAIL" or "Apo2L" are used to refer generally to forms of the Apo2L/TRAIL which include monomer, dimer or trimer forms of the polypeptide. All numbering of amino acid residues referred to in the Apo2L sequence use the numbering according to Figure 1 (SEQ ID NO:1), unless specifically stated otherwise. For instance, "D203" or "Asp203" refers to the aspartic acid residue at position 203 in the sequence provided in Figure 1 (SEQ ID NO:1).

The term "Apo2L/TRAIL extracellular domain" or "Apo2L/TRAIL ECD" refers to a form of Apo2L/TRAIL which is essentially free of transmembrane and cytoplasmic domains. Ordinarily, the ECD will have less than 1% of such transmembrane and cytoplasmic domains, and preferably, will have less than 0.5% of such domains. It will be understood that any transmembrane domain(s) identified for the polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified. In preferred embodiments, the ECD will consist of a soluble, extracellular domain sequence of the polypeptide which is free of the transmembrane and cytoplasmic or intracellular domains (and is not membrane bound). Particular extracellular domain sequences of Apo-2L/TRAIL are described in PCT Publication Nos. WO97/01633 and WO97/25428.

The term "Apo2L/TRAIL monomer" or "Apo2L monomer" refers to a covalent chain of an extracellular domain sequence of Apo2L.

The term "Apo2L/TRAIL dimer" or "Apo2L dimer" refers to two Apo-2L monomers joined in a covalent linkage via a disulfide bond. The term as used herein includes free standing Apo2L dimers and Apo2L dimers that are within trimeric forms of Apo2L (i.e., associated with another, third Apo2L monomer).

The term "Apo2L/TRAIL trimer" or "Apo2L trimer" refers to three Apo2L monomers that are non-covalently associated.

The term "Apo2L/TRAIL aggregate" is used to refer to self-associated higher oligomeric forms of Apo2L/TRAIL, such as Apo2L/TRAIL trimers, which form, for instance, hexameric and nanomeric forms of Apo2L/TRAIL. Determination of the presence and quantity of Apo2L/TRAIL monomer, dimer, or trimer (or other aggregates) may be made using methods and assays known in the art (and using commercially available materials), such as native size exclusion HPLC ("SEC"), denaturing size exclusion using sodium dodecyl sulphate ("SDS-SEC"), reverse phase HPLC and capillary electrophoresis.

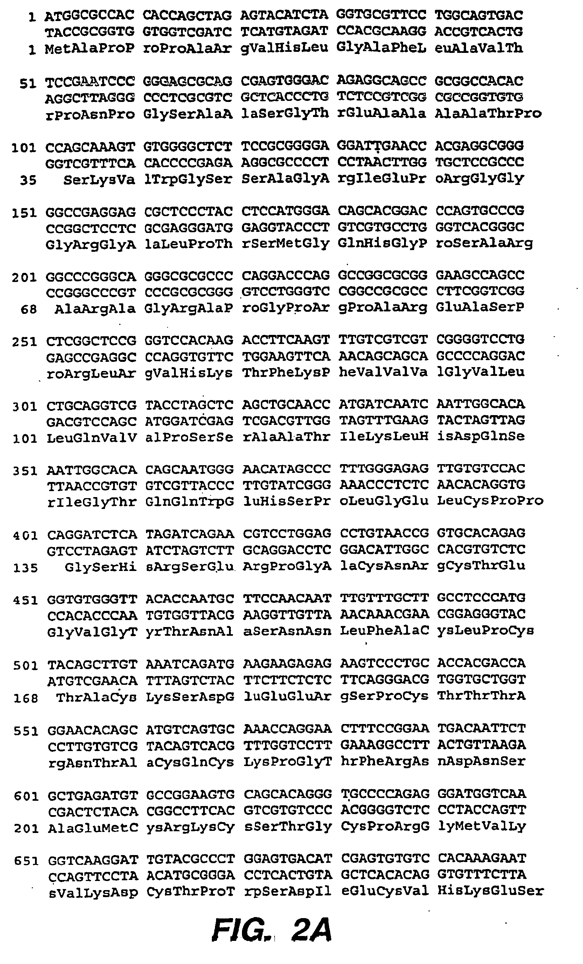

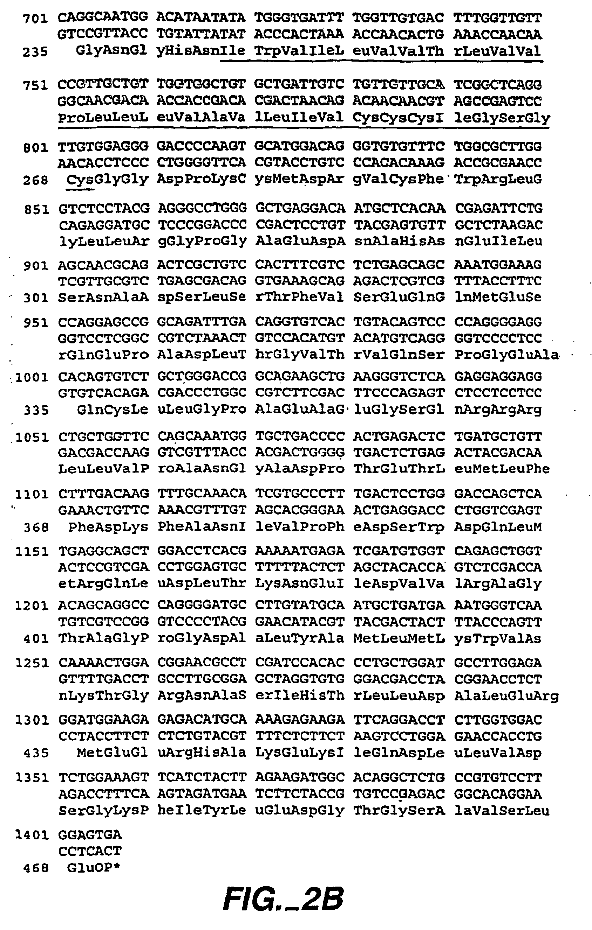

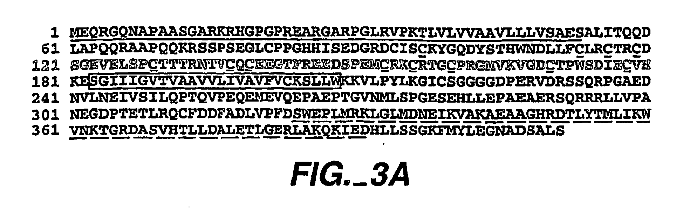

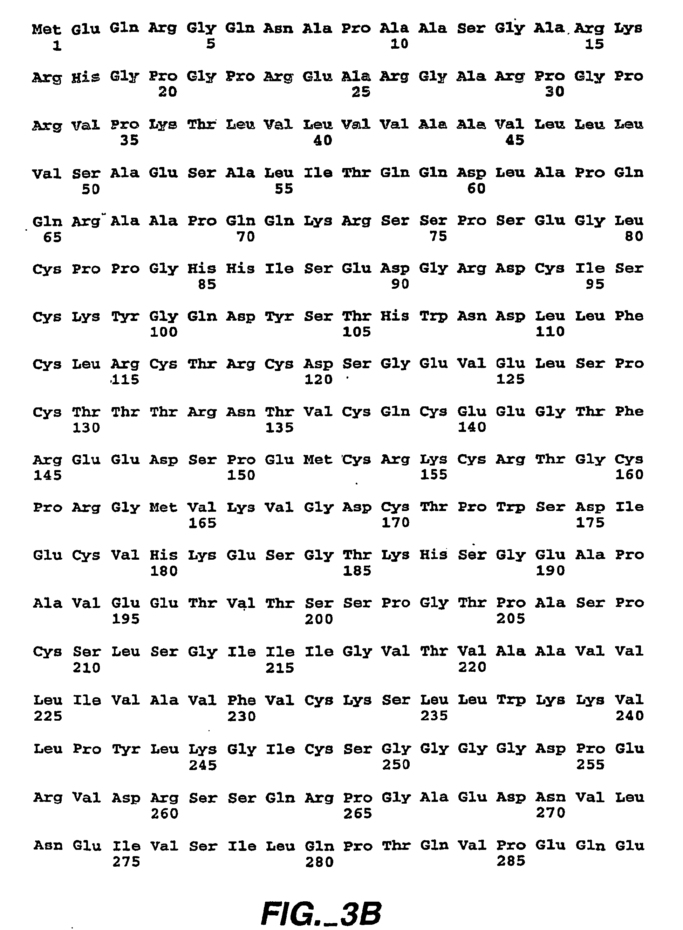

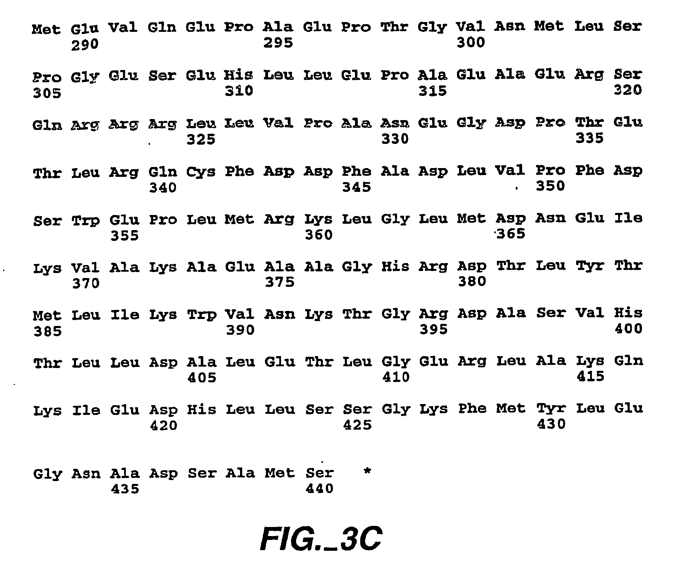

"Apo-2 ligand receptor" includes the receptors referred to in the art as "DR4" and "DR5" whose polynucleotide and polypeptide sequences are shown in Figures 2 and 3 respectively. Pan et al. have described the TNF receptor family member referred to as "DR4" (Pan et al., Science, 276:111-113 (1997); see also WO98/32856 published July 30, 1998). The DR4 receptor was reported to contain a cytoplasmic death domain capable of engaging the cell suicide apparatus. Pan et al. disclose that DR4 is believed to be a receptor for the ligand known as Apo2L/TRAIL. Sheridan et al., Science, 277:818-821 (1997) and Pan et al., Science, 277:815-818 (1997) described another receptor for Apo2L/TRAIL (see also, WO98/51793 published November 19, 1998; WO98/41629 published September 24, 1998). This receptor is referred to as DR5 (the receptor has also been alternatively referred to as Apo-2; TRAIL-R, TR6, Tango-63, hAP08, TRICK2 or KILLER; Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/1179 published March 11, 1999). Like DR4, DR5 is reported to contain a cytoplasmic death domain and be capable of signaling apoptosis. As described above, other receptors for Apo-2L include DcR1, DcR2, and OPG (see, Sheridan et al., supra; Marsters et al., supra; and Simonet et al., supra). The term "Apo-2L receptor" when used herein encompasses native sequence receptor and receptor variants. These terms encompass Apo-2L receptor expressed in a variety of mammals, including humans. Apo-2L receptor may be endogenously expressed as occurs naturally in a variety of human tissue lineages, or may be expressed by recombinant or synthetic methods. A "native sequence Apo-2L receptor" comprises a polypeptide having the same amino acid sequence as an Apo-2L receptor derived from nature. Thus, a native sequence Apo-2L receptor can have the amino acid sequence of naturally-occurring Apo-2L receptor from any mammal. Such native sequence Apo-2L receptor can be isolated from nature or can be produced by recombinant or synthetic means. The term "native sequence Apo-2L receptor" specifically encompasses naturally-occurring truncated or secreted forms of the receptor (*e.g.*, a soluble form containing, for instance, an extracellular domain sequence), naturally-occurring variant forms (*e.g.*, alternatively spliced forms) and naturally-occurring allelic variants. Receptor variants may include fragments or deletion mutants of the native sequence Apo-2L receptor.

The term "antagonist" is used in the broadest sense, and includes any molecule that partially or fully blocks, inhibits, or neutralizes one or more biological activities of Apo2L/TRAIL, DR4 or DR5, *in vitro*, *in situ*, or *in vivo.* Examples of such biological activities of Apo2L/TRAIL, DR4 or DR5 include binding of Apo2L/TRAIL to DR4 or DR5, induction of apoptosis as well as those further reported in the literature. An antagonist may function in a direct or indirect manner. For instance, the antagonist may function to partially or fully block, inhibit or neutralize one or more biological activities of Apo2L/TRAIL, *in vitro*, *in situ,* or *in vivo* as a result of its direct binding to DR4 or DR5. The antagonist may also function indirectly to partially or fully block, inhibit or neutralize one or more biological activities of Apo2L/TRAIL, DR4 or DR5, *in vitro*, *in situ,* or *in vivo* as a result of, *e.g.*, blocking or inhibiting another effector molecule. The antagonist molecule may comprise a "dual" antagonist activity wherein the molecule is capable of partially or fully blocking, inhibiting or neutralizing a biological activity of Apo2L/TRAIL, DR4 or DR5.

The term "agonist" is used in the broadest sense, and includes any molecule that partially or fully enhances, stimulates or activates one or more biological activities of Apo2L/TRAIL, DR4 or DR5, *in vitro*, *in situ*, or *in vivo.* Examples of such biological activities binding of Apo2L/TRAIL to DR4 or DR5, apoptosis as well as those further reported in the literature. An agonist may function in a direct or indirect manner. For instance, the agonist may function to partially or fully enhance, stimulate or activate one or more biological activities of DR4 or DR5, *in vitro*, *in situ,* or *in vivo* as a result of its direct binding to DR4 or DR5, which causes receptor activation or signal transduction. The agonist may also function indirectly to partially or fully enhance, stimulate or activate one or more biological activities of DR4 or DR5, *in vitro, in situ*, or *in vivo* as a result of, e.g., stimulating another effector molecule which then causes DR4 or DR5 activation or signal transduction. It is contemplated that an agonist may act as an enhancer molecule which functions indirectly to enhance or increase DR4 or DR5 activation or activity. For instance, the agonist may enhance activity of endogenous Apo-2L in a mammal. This could be accomplished, for example, by pre-complexing DR4 or DR5 or by stabilizing complexes of the respective ligand with the DR4 or DR5 receptor (such as stabilizing native complex formed between Apo-2L and DR4 or DR5).

The term "tagged" when used herein refers to a chimeric molecule comprising a Apo-2L receptor binding peptide or alternatively, Apo2L/TRAIL, or a portion thereof, fused to a "tag polypeptide". The tag polypeptide has enough residues to provide an epitope against which an antibody can be made or to provide some other function, such as the ability to oligomerize (e.g. as occurs with peptides having leucine zipper domains), yet is short enough such that it generally does not interfere with activity of the Apo-2L receptor binding peptide. The tag polypeptide preferably also is fairly unique so that a tag-specific antibody does not substantially cross-react with other epitopes. Suitable tag polypeptides generally have at least six amino acid residues and usually between about 8 to about 50 amino acid residues (preferably, between about 10 to about 20 residues).

The term "divalent metal ion" refers to a metal ion having two positive charges. Examples of divalent metal ions include but are not limited to zinc, cobalt, nickel, cadmium, magnesium, and manganese. Particular forms of such metals that may be employed include salt forms (e.g., pharmaceutically acceptable salt forms), such as chloride, acetate, carbonate, citrate and sulfate forms of the above mentioned divalent metal ions. Optionally, a divalent metal ion for use in the present invention is zinc, and preferably, the salt form, zinc sulfate or zinc chloride.

"Isolated," when used to describe the various peptides or proteins disclosed herein, means peptide or protein that has been identified and separated and/or recovered from a component of its natural environment. Contaminant components of its natural environment are materials that would typically interfere with diagnostic or therapeutic uses for the peptide or protein, and may include enzymes, hormones, and other proteinaceous or non-proteinaceous solutes. In preferred embodiments, the peptide or protein will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain, or (3) to homogeneity by mass spectroscopic or peptide mapping techniques. Isolated material includes peptide or protein in situ within recombinant cells, since at least one component of its natural environment will not be present. Ordinarily, however, isolated peptide or protein will be prepared by at least one purification step.

"Percent (%) amino acid sequence identity" with respect to the sequences identified herein is defined as the percentage of amino acid residues in a candidate sequence that are identical with the amino acid residues in the reference sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art can determine appropriate parameters for measuring alignment, including assigning algorithms needed to achieve maximal alignment over the full-length sequences being compared. For purposes herein, percent amino acid identity values can be obtained using the sequence comparison computer program, ALIGN-2, which was authored by Genentech, Inc. and the source code of which has been filed with user documentation in the US Copyright Office, Washington, DC, 20559, registered under the US Copyright Registration No. TXU510087. The ALIGN-2 program is publicly available through Genentech, Inc., South San Francisco, CA. All sequence comparison parameters are set by the ALIGN-2 program and do not vary.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired identity between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"High stringency conditions", as defined herein, are identified by those that: (1) employ low ionic strength and high temperature for washing; 0.015 M sodium chloride/0.0015 M sodium citrate/0.1% sodium dodecyl sulfate at 50°C; (2) employ during hybridization a denaturing agent; 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors; platelet-growth factor; transforming growth factors (TGFs) such as TGF-α and TNF-β; insulin-like growth factor-I and -II; erythropoietin (EPO) ; osteoinductive factors; interferons such as interferon-α, -β, and - gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-2, IL-3, IL-4, IL-5, IL-6, IL-7, IL-8, IL-9, IL-11, IL-12, IL-13, IL-17; and other polypeptide factors including LIF and kit ligand (KL). As used herein, the term cytokine includes proteins from natural sources or from recombinant cell culture and biologically active equivalents of the native sequence cytokines.

The term "cytotoxic agent" as used herein refers to a substance that inhibits or prevents the function of cells and/or causes destruction of cells. The term is intended to include radioactive isotopes (*e.g.*, I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include alkylating agents such as thiotepa and cyclosphosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (*e.g.* calicheamicin, especially calicheamicin gamma1I and calicheamicin phiI1, see, *e.g.*, Agnew, Chem Intl. Ed. Engl., 33:183-186 (1994); dynemicin, including dynemicin A; bisphosphonates, such as clodronate; an esperamicin; as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (Adriamycin^{™}) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{®}; razoxane; rhizoxin; sizofiran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, *e.g.* paclitaxel (TAXOL^{°}. Bristol-Myers Squibb Oncology, Princeton, NJ) and doxetaxel (TAXOTERE^{®}, Rhône-Poulenc Rorer, Antony, France); chlorambucil; gemcitabine (Gemzar^{™}) ; 6-thioguanine; mercaptopurine; methotrexate; platinum analogs such as cisplatin and carboplatin; vinblastine; platinum; etoposide (VP-16); ifosfamide; mitoxantrone; vincristine; vinorelbine (Navelbine^{™}); novantrone; teniposide; edatrexate; daunomycin; aminopterin; xeloda; ibandronate; CPT-11; topoisomerase inhibitor RFS 2000; difluoromethylornithine (DMFO) ; retinoids such as retinoic acid; capecitabine; and pharmaceutically acceptable salts, acids or derivatives of any of the above. Also included in this definition are anti-hormonal agents that act to regulate or inhibit hormone action on tumors such as antiestrogens and selective estrogen receptor modulators (SERMs), including, for example, tamoxifen (including Nolvadex^{™}), raloxifene, droloxifene, 4-hydroxytamoxifen, trioxifene, keoxifene, LY117018, onapristone, and toremifene (Fareston^{™}); aromatase inhibitors that inhibit the enzyme aromatase, which regulates estrogen production in the adrenal glands, such as, for example, 4(5)-imidazoles, aminoglutethimide, megestrol acetate (Megace^{™}), exemestane, formestane, fadrozole, vorozole (Rivisor^{™}), letrozole (Femara^{™}), and anastrozole (Arimidex^{™}); and anti-androgens such as flutamide, nilutamide, bicalutamide, leuprolide, and goserelin; and pharmaceutically acceptable salts, acids or derivatives of any of the above.

A "growth inhibitory agent" when used herein refers to a compound or composition which inhibits growth of a cell, especially cancer cell overexpressing any of the genes identified herein, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

"Biologically active" or "biological activity" for the purposes herein means (a) having the ability to induce or stimulate or inhibit apoptosis in at least one type of mammalian cancer cell or virally-infected cell *in vivo* or *ex vivo*, either alone as a single agent or in combination with another agent such as a chemotherapeutic agent (b) capable of binding and/or stimulating a receptor for Apo2L/TRAIL (such receptors may include the DR4 receptor, DR5 receptor, OPG, DcR1 receptor, and DcR2 receptor); or (c) having some activity of a native or naturally-occurring Apo2L/TRAIL polypeptide. Assays for determining biological activity can be conducted using methods known in the art, such as DNA fragmentation (see, *e.g.*, Marsters et al., Curr. Biology, 6: 1669 (1996)), caspase inactivation, DR4 binding, DR5 binding (see, e.g., WO 98/51793, published Nov. 19, 1998), DcR1 binding (see, e.g., WO 98/58062, published Dec. 23, 1998), DcR2 binding (see, e.g., WO 99/10484, published March 4, 1999) as well as the assays described in PCT Publication Nos. WO97/01633, WO97/25428, WO 01/00832, and WO 01/22987.

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays (such as Alamar blue assays or MTT assays), FACS analysis, caspase activation, DNA fragmentation (see, for example, Nicoletti et al., J. Immunol. Methods, 139:271-279 (1991), and poly-ADP ribose polymerase, "PARP", cleavage assays known in the art.

As used herein, the term "disorder" in general refers to any condition that would benefit from treatment with the compositions described herein, including any disease or disorder that can be treated by effective amounts of an Apo-2L receptor binding peptide. This includes chronic and acute disorders, as well as those pathological conditions which predispose the mammal to the disorder in question. Non-limiting examples of disorders to be treated herein include benign and malignant cancers; inflammatory, angiogenic, and immunologic disorders, autoimmune disorders, arthritis (including rheumatoid arthritis), multiple sclerosis, and HIV/AIDS.

The terms "cancer", "cancerous", or "malignant" refer to or describe the physiological condition in mammals that is typically characterized by unregulated cell growth. Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, gastrointestinal cancer, renal cancer, ovarian cancer, liver cancer, lymphoblastic leukemia, lymphocytic leukemia, colorectal cancer, endometrial cancer, kidney cancer, prostate cancer, thyroid cancer, neuroblastoma, pancreatic cancer, glioblastoma multiforme, cervical cancer, brain cancer, stomach cancer, bladder cancer, hepatoma, breast cancer, colon carcinoma, and head and neck cancer.

The term "immune related disease" means a disease in which a component of the immune system of a mammal causes, mediates or otherwise contributes to morbidity in the mammal. Also included are diseases in which stimulation or intervention of the immune response has an ameliorative effect on progression of the disease. Included within this term are autoimmune diseases, immune-mediated inflammatory diseases, non-immune-mediated inflammatory diseases, infectious diseases, and immunodeficiency diseases. Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory and fibrotic lung diseases such as inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease. Infectious diseases include AIDS (HIV infection), hepatitis A, B, C, D, and E, bacterial infections, fungal infections, protozoal infections and parasitic infections.

"Autoimmune disease" is used herein in a broad, general sense to refer to disorders or conditions in mammals in which destruction of normal or healthy tissue arises from humoral or cellular immune responses of the individual mammal to his or her own tissue constituents. Examples include, but are not limited to, lupus erythematous, thyroiditis, rheumatoid arthritis, psoriasis, multiple sclerosis, autoimmune diabetes, and inflammatory bowel disease (IBD).

The terms "treating", "treatment" and "therapy" as used herein refer to curative therapy, prophylactic therapy, and preventative therapy. Consecutive treatment or administration refers to treatment on at least a daily basis without interruption in treatment by one or more days. Intermittent treatment or administration, or treatment or administration in an intermittent fashion, refers to treatment that is not consecutive, but rather cyclic in nature.

The term "mammal" as used herein refers to any mammal classified as a mammal, including humans, cows, horses, dogs and cats. In a preferred embodiment of the invention, the mammal is a human.

### B. EXEMPLARY MATERIALS AND METHODS OF THE INVENTION

The invention described herein relates to peptides that bind to Apo-2L receptors. Optionally the peptide is an antagonist which inhibits the interaction of Apo-2L with DR5. Alternatively, the peptide compound is an agonist of DR5 signalling activity. Optionally the peptide compound is an antagonist which inhibits the interaction of Apo-2L with DR4. Alternatively, the peptide compound is an agonist of DR4 signalling activity. Preferably, the peptide has about 10 to about 25 amino acid residues, or about 15 to about 20 residues, and more preferably, 12 to 20 amino acid residues. More preferably, the peptide has a cysteine residue at position 1, 2, 3, 4, 5, 6 or 7 numbered from its N-terminus. Still more preferably, the peptide additionally has a valine, glutamic acid, or glycine residue N-terminal to the N-terminal cysteine residue. Even more preferably, the peptide is such that the residue N-terminal to the N-terminal cysteine residue is a valine residue. Still more preferably, the peptide has a motif comprising cysteine-X₁-X₂-X₃-cysteine. Preferably, X₁ is either a methionine or a leucine residue. Preferably X₂ is a threonine or alanine residue. Preferably X₃ is a serine, methionine, leucine or glutamic acid residue. This peptide even more preferably has a leucine residue at position 2 numbered from its C-terminus.

The peptides provided by this invention can be made using techniques known in the art, such as by chemical synthesis or by employing recombinant technology. Chemical synthesis, especially solid phase synthesis, is preferred for relatively short (*e.g.*, less than 50 residues) peptides or those containing unnatural or unusual amino acids such as D-Tyr, Ornithine, amino adipic acid, and the like. Recombinant procedures are preferred for longer polypeptides. When recombinant procedures are selected, a synthetic gene may be constructed de novo or a natural gene may be mutated by, for example, cassette mutagenesis. Set forth below are exemplary general recombinant procedures.

Using DR4 or DR5 receptors and their respective amino acid sequences, for example, an Apo-2L receptor binding peptide antagonist or agonist may be identified using the methods disclosed herein. Optionally, the Apo-2L receptor binding peptide can be produced using methods known in the art such as peptide synthesis or recombinant DNA techniques. The DNA. techniques contemplate, in simplified form, taking the gene, either natural or synthetic, encoding the peptide; inserting it into an appropriate vector; inserting the vector into an appropriate host cell; culturing the host cell to cause expression of the gene; and recovering or isolating the peptide produced thereby. Preferably, the recovered peptide is then purified to a suitable degree.

Somewhat more particularly, the DNA sequence encoding a peptidyl Apo-2L receptor binding peptide is cloned and manipulated so that it may be expressed in a convenient host. DNA encoding parent polypeptides can be obtained from a genomic library, from cDNA derived from mRNA from cells expressing the peptide, or by synthetically constructing the DNA sequence (Sambrook et al., Molecular Cloning: A Laboratory Manual (2d ed.), Cold Spring Harbor Laboratory, N.Y., 1989).

The parent DNA is then inserted into an appropriate plasmid or vector which is used to transform a host cell. In general, plasmid vectors containing replication and control sequences which are derived from species compatible with the host cell are used in connection with those hosts. The vector ordinarily carries a replication site, as well as sequences which encode proteins or peptides that are capable of providing phenotypic selection in transformed cells.

For example, *E. coli* may be transformed using pBR322, a plasmid derived from an *E. coli* species. Mandel et al., J. Mol. Biol. 53: 154 (1970). Plasmid pBR322 contains genes for ampicillin and tetracycline resistance, and thus provides easy means for selection. Other vectors include different features such as different promoters, which are often important in expression. For example, plasmids pKK223-3, pDR720, and pPL-lambda represent expression vectors with the *tac*, *trp,* or *P_{L}* promoters that are currently available (Pharmacia Biotechnology).

Other preferred vectors can be constructed using standard techniques by combining the relevant traits of the vectors described above. Relevant traits include the promoter, the ribosome binding site, the decorsin or ornatin gene or gene fusion (the Z domain of protein A and decorsin or ornatin and its linker), the antibiotic resistance markers, and the appropriate origins of replication.

The host cell may be prokaryotic or eukaryotic. Prokaryotes are preferred for cloning and expressing DNA sequences to produce parent polypeptide, segment-substituted peptides, residue-substituted peptides, and peptide variants. For example, *E. coli* K12 strain 294 (ATCC No. 31446) may be used as well as *E. coli* B, *E. coli* X1776 (ATCC No. 31537), and *E. coli* c600 and c600hfl, *E. coli* W3110 (F-, gamma-, prototrophic/ATCC No. 27325), bacilli such as *Bacillus subtilis,* and other enterobacteriaceae such as *Salmonella typhimurium* or *Serratia marcesans*, and various *Pseudomonas* species. The preferred prokaryote is *E. coli* W3110 (ATCC 27325). When expressed by prokaryotes the peptides typically contain an N-terminal methionine or a formyl methionine and are not glycosylated. In the case of fusion proteins, the N-terminal methionine or formyl methionine resides on the amino terminus of the fusion protein or the signal sequence of the fusion protein. These examples are, of course, intended to be illustrative rather than limiting.

In addition to prokaryotes, eukaryotic organisms, such as yeast cultures, or cells derived from multicellular organisms may be used. In principle, any such cell culture is workable. However, interest has been greatest in vertebrate cells, and propagation of vertebrate cells in culture (tissue culture) has become a reproducible procedure. Tissue Culture, Academic Press, Kruse and Patterson, editors (1973). Examples of such useful host cell lines are VERO and HeLa cells, Chinese Hamster Ovary (CHO) cell lines, W138, 293, BHK, COS-7 and MDCK cell lines.

A variation on the above procedures contemplates the use of gene fusions, wherein the gene encoding the desired peptide is associated, in the vector, with a gene encoding another protein or a fragment of another protein. This results in the desired peptide being produced by the host cell as a fusion with another protein or peptide. The "other" protein or peptide is often a protein or peptide which can be secreted by the cell, making it possible to isolate and purify the desired peptide from the culture medium and eliminating the necessity of destroying the host cells which arises when the desired peptide remains inside the cell. Alternatively, the fusion protein can be expressed intracellularly. It is useful to use fusion proteins that are highly expressed.

In a preferred embodiment, the peptide can be fused with an immunoglobulin or a particular region of an immunoglobulin such as the Fc region of an IgG molecule. Such fusions can be generated for example in order to extend the in vivo plasma half-life of a desired peptide composition. In a typical embodiment, the immunoglobulin fusion includes the hinge, CH2 and CH3, or the hinge, CHI, CH2 and CH3 regions of an IgG1 molecule. For the production of immunoglobulin fusions see, e.g., U.S. Patent No. 5,428,130 which is incorporated herein by reference.

The use of gene fusions, though not essential, can facilitate the expression of heterologous peptides in *E. coli* as well as the subsequent purification of those gene products. Harris, in Genetic Engineering, Williamson, R., Ed. (Academic Press, London, Vol. 4, 1983), p. 127; Ljungquist et al., Eur. J. Biochem., 186: 557-561 (1989) and Ljungquist et al., Eur. J. Biochem., 186: 563-569 (1989). Protein A fusions are often used because the binding of protein A, or more specifically the Z domain of protein A, to IgG provides an "affinity handle" for the purification of the fused protein. It has also been shown that many heterologous proteins are degraded when expressed directly in *E. coli*, but are stable when expressed as fusion proteins. Marston, Biochem J., 240: 1 (1986). The use of gene fusions can also facilitate the formation of oligomeric complexes such as Apo-2L receptor binding peptide dimers, trimers and tetrameters. Preferably the heterologous peptide sequence comprises a leucine zipper domain, as discussed in Example 3 below.

Fusion proteins can be cleaved using chemicals, such as cyanogen bromide, which cleaves at a methionine, or hydroxylamine, which cleaves between an Asn and Gly residue. Using standard recombinant DNA methodology, the nucleotide base pairs encoding these amino acids may be inserted just prior to the 5' end of the gene encoding the desired peptide.

Alternatively, one can employ proteolytic cleavage of fusion protein. Carter, in Protein Purification: From Molecular Mechanisms to Large-Scale Processes, Ladisch et al., eds. (American Chemical Society Symposium Series No. 427, 1990), Ch 13, pages 181-193.

Proteases such as Factor Xa, thrombin, and subtilisin or its mutants, and a number of others have been successfully used to cleave fusion proteins. Typically, a peptide linker that is amenable to cleavage by the protease used is inserted between the "other" protein (*e.g.*, the Z domain of protein A) and the desired peptide. Using recombinant DNA methodology, the nucleotide base pairs encoding the linker are inserted between the genes or gene fragments coding for the other proteins. Proteolytic cleavage of the partially purified fusion protein containing the correct linker can then be carried out on either the native fusion protein, or the reduced or denatured fusion protein.

The peptide may or may not be properly folded when expressed as a fusion protein. Also, the specific peptide linker containing the cleavage site may or may not be accessible to the protease. These factors determine whether the fusion protein must be denatured and refolded, and if so, whether these procedures are employed before or after cleavage.

When denaturing and refolding are needed, typically the peptide is treated with a chaotrope, such a guanidine HCl, and is then treated with a redox buffer, containing, for example, reduced and oxidized dithiothreitol or glutathione at the appropriate ratios, pH, and temperature, such that the peptide is refolded to its native structure.

When peptides are not prepared using recombinant DNA technology, they are preferably prepared using solid-phase synthesis, such as that generally described by Merrifield, J. Am. Chem. Soc., 85: 2149 (1963), although other equivalent chemical syntheses known in the art are employable. Solid-phase synthesis is initiated from the C-terminus of the peptide by coupling a protected α-amino acid to a suitable resin. Such a starting material can be prepared by attaching an α-amino-protected amino acid by an ester linkage to a chloromethylated resin or a hydroxymethyl resin, or by an amide bond to a BHA resin or MBHA resin. The preparation of the hydroxymethyl resin is described by Bodansky et al., Chem. Ind. (London), 38: 1597-1598 (1966). Chloromethylated resins are commercially available from BioRad Laboratories, Richmond, CA and from Lab. Systems, Inc. The preparation of such a resin is described by Stewart et al., "Solid Phase Peptide Synthesis" (Freeman & Co., San Francisco 1969), Chapter 1, pp. 1-6. BHA and MBHA resin supports are commercially available and are generally used only when the desired polypeptide being synthesized has an unsubstituted amide at the C-terminus. The amino acids are coupled to the peptide chain using techniques well known in the art for the formation of peptide bonds. One method involves converting the amino acid to a derivative that will render the carboxyl group more susceptible to reaction with the free N-terminal amino group of the peptide fragment. For example, the amino acid can be converted to a mixed anhydride by reaction of a protected amino acid with ethylchloroformate, phenyl chloroformate, sec-butyl chloroformate, isobutyl chloroformate, pivaloyl chloride or like acid chlorides. Alternatively, the amino acid can be converted to an active ester such as a 2,4,5-trichlorophenyl ester, a pentachlorophenyl ester, a pentafluorophenyl ester, a p-nitrophenyl ester, a N-hydroxysuccinimide ester, or an ester formed from 1-hydroxybenzotriazole.

Another coupling method involves use of a suitable coupling agent such as N,N'-dicyclohexylcarbodiimide or N,N'-diisopropyl-carbodiimide. Other appropriate coupling agents, apparent to those skilled in the art, are disclosed in E. Gross & J. Meienhofer, The Peptides: Analysis, Structure, Biology, Vol. I: Major Methods of Peptide. Bond Formation (Academic Press, New York, 1979).

It should be recognized that the α-amino group of each amino acid employed in the peptide synthesis must be protected during the coupling reaction to prevent side reactions involving their active α-amino function. It should also be recognized that certain amino acids contain reactive side-chain functional groups (*e.g.*, sulfhydryl, amino, carboxyl, and hydroxyl) and that such functional groups must also be protected with suitable protecting groups to prevent a chemical reaction from occurring at that site during both the initial and subsequent coupling steps. Suitable protecting groups, known in the art, are described in Gross and Meienhofer, The Peptides: Analysis, Structure, Biology, Vol.3: "Protection of Functional Groups in Peptide Synthesis" (Academic Press, New York, 1981).

In the selection of a particular side-chain protecting group to be used in synthesizing the peptides, the following general rules are followed. An α-amino protecting group (a) must render the α-amino function inert under the conditions employed in the coupling reaction, (b) must be readily removable after the coupling reaction under conditions that will not remove side-chain protecting groups and will not alter the structure of the peptide fragment, and (c) must eliminate the possibility of racemization upon activation immediately prior to coupling. A side-chain protecting group (a) must render the side chain functional group inert under the conditions employed in the coupling reaction, (b) must be stable under the conditions employed in removing the α-amino protecting group, and (c) must be readily removable upon completion of the desired amino acid peptide under reaction conditions that will not alter the structure of the peptide chain.

It will be apparent to those skilled in the art that the protecting groups known to be useful for peptide synthesis will vary in reactivity with the agents employed for their removal. For example, certain protecting groups such as triphenylmethyl and 2-(p-biphenylyl)isopropyloxycarbonyl are very labile and can be cleaved under mild acid conditions. Other protecting groups, such as t-butyloxycarbonyl (BOC), t-amyloxycarbonyl, adamantyl-oxycarbonyl, and p-methoxybenzyloxycarbonyl are less labile and require moderately strong acids, such as trifluoroacetic, hydrochloric, or boron trifluoride in acetic acid, for their removal. Still other protecting groups, such as benzyloxycarbonyl (CBZ or Z), halobenzyloxycarbonyl, p-nitrobenzyloxycarbonyl cycloalkyloxycarbonyl, and isopropyloxycarbonyl, are even less labile and require stronger acids, such as hydrogen fluoride, hydrogen bromide, or boron trifluoroacetate in trifluoroacetic acid, for their removal. Among the classes of useful amino acid protecting groups are included:
(1) for an α-amino group, (a) aromatic urethane-type protecting groups, such as fluorenylmethyloxycarbonyl (FMOC) CBZ, and substituted CBZ, such as, *e.g.*, p-chlorobenzyloxycarbonyl, p-6-nitrobenzyloxycarbonyl, p-bromobenzyloxycarbonyl, and p-methoxybenzyloxycarbonyl, o-chlorobenzyloxycarbonyl, 2,4-dichlorobenzyloxycarbonyl, 2,6-dichlorobenzyloxycarbonyl, and the like; (b) aliphatic urethane-type protecting groups, such as BOC, t-amyloxycarbonyl, isopropyloxycarbonyl, 2-(p-biphenylyl)-isopropyloxycarbonyl, allyloxycarbonyl and the like; (c) cycloalkyl urethane-type protecting groups, such as cyclopentyloxycarbonyl, adamantyloxycarbonyl, and cyclohexyloxycarbonyl; and d) allyloxycarbonyl. The preferred α-amino protecting groups are BOC or FMOC.
(2) for the side chain amino group present in Lys, protection may be by any of the groups mentioned above in (1) such as BOC, p-chlorobenzyloxycarbonyl, etc.
(3) for the guanidino group of Arg, protection may be by nitro, tosyl, CBZ, adamantyloxycarbonyl, 2,2,5,7,8-pentamethylchroman-6-sulfonyl or 2,3,6-trimethyl-4-methoxyphenylsulfonyl, or BOC.
(4) for the hydroxyl group of Ser, Thr, or Tyr, protection may be, for example, by C1-C4 alkyl, such as t-butyl; benzyl (BZL); substituted BZL, such as p-methoxybenzyl, p-nitrobenzyl, p-chlorobenzyl, o-chlorobenzyl, and 2,6-dichlorobenzyl.
(5) for the carboxyl group of Asp or Glu, protection may be, for example, by esterification using groups such as BZL, t-butyl, cyclohexyl, cyclopentyl, and the like.
(6) for the imidazole nitrogen of His, the tosyl moiety is suitably employed.
(7) for the phenolic hydroxyl group of Tyr, a protecting group such as tetrahydropyranyl, tert-butyl, trityl, BZL, chlorobenzyl, 4-bromobenzyl, or 2,6-dichlorobenzyl is suitably employed. The preferred protecting group is 2,6-dichlorobenzyl.
(8) for the side chain amino group of Asn or Gln, xanthyl (Xan) is preferably employed.
(9) for Met, the amino acid is preferably left unprotected.
(10) for the thio group of Cys, p-methoxybenzyl is typically employed.

The C-terminal amino acid, *e.g.*, Lys, is protected at the N-amino position by an appropriately selected protecting group, in the case of Lys, BOC. The BOC-Lys-OH can be first coupled to the benzyhydrylamine or chloromethylated resin according to the procedure set forth in Horiki et al., Chemistry Letters, 165-168 1978) or using isopropylcarbodiimide at about 25°C for 2 hours with stirring. Following the coupling of the BOC-protected amino acid to the resin support, the α-amino protecting group is removed, as by using trifluoroacetic acid (TFA) in methylene chloride or TFA alone. The deprotection is carried out at a temperature between about 0°C and room temperature. Other standard cleaving reagents, such as HCl in dioxane, and conditions for removal of specific α-amino protecting groups are described in the literature.

After removal of the α-amino protecting group, the remaining α-amino and side-chain protected amino acids are coupled stepwise within the desired order. As an alternative to adding each amino acid separately in the synthesis, some may be coupled to one another prior to addition to the solid-phase synthesizer. The selection of an appropriate coupling reagent is within the skill of the art. Particularly suitable as a coupling reagent is N,N'-dicyclohexyl carbodiimide or diisopropylcarbodiimide.

Each protected amino acid or amino acid sequence is introduced into the solid-phase reactor in excess, and the coupling is suitably carried out in a medium of dimethylformamide (DMF) or CH₂Cl₂ or mixtures thereof. If incomplete coupling occurs, the coupling procedure is repeated before removal of the N-amino protecting group prior to the coupling of the next amino acid. The success of the coupling reaction at each stage of the synthesis may be monitored. A preferred method of monitoring the synthesis is by the ninhydrin reaction, as described by Kaiser et al., Anal. Biochem, 34: 595 (1970). The coupling reactions can be performed automatically using well known methods, for example, a BIOSEARCH 9500^{™} peptide synthesizer.

Upon completion of the desired peptide sequence, the protected peptide must be cleaved from the resin support, and all protecting groups must be removed. The cleavage reaction and removal of the protecting groups is suitably accomplished simultaneously or stepwise. When the resin support is a chloro-methylated polystyrene resin, the bond anchoring the peptide to the resin is an ester linkage formed between the free carboxyl group of the C-terminal residue and one of the many chloromethyl groups present on the resin matrix. It will be appreciated that the anchoring bond can be cleaved by reagents that are known to be capable of breaking an ester linkage and of penetrating the resin matrix.

One especially convenient method is by treatment with liquid anhydrous hydrogen fluoride. This reagent not only will cleave the peptide from the resin but also will remove all protecting groups. Hence, use of this reagent will directly afford the fully deprotected peptide. When the chloromethylated resin is used, hydrogen fluoride treatment results in the formation of the free peptide acids. When the benzhydrylamine resin is used, hydrogen fluoride treatment results directly in the free peptide amines. Reaction with hydrogen fluoride in the presence of anisole and dimethylsulfide at 0°C for one hour will simultaneously remove the side-chain protecting groups and release the peptide from the resin.

When it is desired to cleave the peptide without removing protecting groups, the protected peptide-resin can undergo methanolysis to yield the protected peptide in which the C-terminal carboxyl group is methylated. The methyl ester is then hydrolyzed under mild alkaline conditions to give the free C-terminal carboxyl group. The protecting groups on the peptide chain then are removed by treatment with a strong acid, such as liquid hydrogen fluoride. A particularly useful technique for methanolysis is that of Moore et al., Peptides, Proc. Fifth Amer. Pept. Symp., M. Goodman and J. Meienhofer, Eds., (John Wiley, N.Y., 1977), p. 518-521, in which the protected peptide-resin is treated with methanol and potassium cyanide in the presence of crown ether.

Another method for cleaving the protected peptide from the resin when the chloromethylated resin is employed is by ammonolysis or by treatment with hydrazine. If desired, the resulting C-terminal amide or hydrazide can be hydrolyzed to the free C-terminal carboxyl moiety, and the protecting groups can be removed conventionally.

It will also be recognized that the protecting group present on the N-terminal α-amino group may be removed preferentially either before or after the protected peptide is cleaved from the support.

Purification of the polypeptides of the invention is typically achieved using conventional procedures such as preparative HPLC (including reversed phase HPLC) or other known chromatographic techniques such as gel permeation, ion exchange, partition chromatography, affinity chromatography (including monoclonal antibody columns) or countercurrent distribution.

The peptides of this invention may be stabilized by polymerization. This may be accomplished by crosslinking monomer chains with polyfunctional crosslinking agents, either directly or indirectly, through multi-functional polymers. Ordinarily, two substantially identical polypeptides are crosslinked at their C- or N-termini using a bifunctional crosslinking agent. The agent is used to crosslink the terminal amino and/or carboxyl groups. Generally, both terminal carboxyl groups or both terminal amino groups are crosslinked to one another, although by selection of the appropriate crosslinking agent the alpha amino of one polypeptide is crosslinked to the terminal carboxyl group of the other polypeptide. Preferably, the polypeptides are substituted at their C-termini with cysteine. Under conditions well known in the art a disulfide bond can be formed between the terminal cysteines, thereby crosslinking the polypeptide chains. For example, disulfide bridges are conveniently formed by metal-catalyzed oxidation of the free cysteines or by nucleophilic substitution of a suitably modified cysteine residue. Selection of the crosslinking agent will depend upon the identities of the reactive side chains of the amino acids present in the polypeptides. For example, disulfide crosslinking would not be preferred if cysteine was present in the polypeptide at additional sites other than the C-terminus. Also within the scope hereof are peptides crosslinked with methylene bridges.

Suitable crosslinking sites on the peptides, aside from the N-terminal amino and C-terminal carboxyl groups, include epsilon amino groups found on lysine residues, as well as amino, imino, carboxyl, sulfhydryl and hydroxyl groups located on the side chains of internal residues of the peptides or residues introduced into flanking sequences. Crosslinking through externally added crosslinking agents is suitably achieved, *e.g.*, using any of a number of reagents familiar to those skilled in the art, for example, via carbodiimide treatment of the polypeptide. Other examples of suitable multi-functional (ordinarily bifunctional) crosslinking agents are found in the literature.

The peptides of this invention also may be conformationally stabilized by cyclization. The peptides ordinarily are cyclized by covalently bonding the N- and C-terminal domains of one peptide to the corresponding domain of another peptide of this invention so as to form cyclo-oligomers containing two or more iterated peptide sequences, each internal peptide having substantially the same sequence. Further, cyclized peptides (whether cyclo-oligomers or cyclo-monomers) are crosslinked to form 1-3 cyclic structures having from 2 to 6 peptides comprised therein. The peptides preferably are not covalently bonded through α-amino and main chain carboxyl groups (head to tail), but rather are crosslinked through the side chains of residues located in the N- and C-terminal domains. The linking sites thus generally will be between the side chains of the residues.

Many suitable methods *per se* are known for preparing mono-or poly-cyclized peptides as contemplated herein. Lys/Asp cyclization has been accomplished using NαBoc-amino acids on solid-phase support with Fmoc/9-fluorenylmethyl (OFm) side-chain protection for Lys/Asp; the process is completed by piperidine treatment followed by cyclization.

Glu and Lys side chains also have been crosslinked in preparing cyclic or bicyclic peptides: the peptide is synthesized by solid phase chemistry on a p-methylbenzhydrylamine resin. The peptide is cleaved from the resin and deprotected. The cyclic peptide is formed using diphenylphosphorylazide in diluted methylformamide. For an alternative procedure, see Schiller et al., Peptide Protein Res., 25: 171-177 (1985). See also U.S. Pat. No. 4,547,489.

Disulfide crosslinked or cyclized peptides are generated by conventional methods. The method of Pelton et al. (J. Med. Chem., 29: 2370-2375 (1986)) is suitable, except that a greater proportion of cyclo-oligomers are produced by conducting the reaction in more concentrated solutions than the dilute reaction mixture described by Pelton et al., for the production of cyclo-monomers. The same chemistry is useful for synthesis of dimers or cyclo-oligomers or cyclo-monomers. Also useful are thiomethylene bridges. Lebl and Hruby, Tetrahedron Letters, 25: 2067-2068 (1984). See also Cody et al., J. Med. Chem., 28: 583 (1985).

The desired cyclic or polymeric peptides are purified by gel filtration followed by reversed-phase high pressure liquid chromatography or other conventional procedures. The peptides are sterile filtered and formulated into conventional pharmacologically acceptable vehicles.

The starting materials required for the processes described herein are known in the literature or can be prepared using known methods and known starting materials.

If in the peptides being created carbon atoms bonded to four nonidentical substituents are asymmetric, then the compounds may exist as diastereoisomers, enantiomers or mixtures thereof. The syntheses described above may employ racemates, enantiomers or diastereomers as starting materials or intermediates. Diastereomeric products resulting from such syntheses may be separated by chromatographic or crystallization methods. Likewise, enantiomeric product mixtures may be separated using the same techniques or by other methods known in the art. Each of the asymmetric carbon atoms, when present, may be in one of two configurations (R or S) and both are within the scope of the present invention.

The peptide compounds described in this invention may be isolated as the free acid or base or converted to salts of various inorganic and organic acids and bases. Such salts are within the scope of this invention. Examples of such salts include ammonium, metal salts like sodium, potassium, calcium and magnesium; salts with organic bases like dicyclohexylamine, N-methyl-D-glucamine and the like; and salts with amino acids like arginine or lysine. Salts with inorganic and organic acids may be likewise prepared, for example, using hydrochloric, hydrobromic, sulfuric, phosphoric, trifluoroacetic, methanesulfonic, malic, maleic, fumaric and the like. Non-toxic and physiologically compatible salts are particularly useful, although other less desirable salts may have use in the processes of isolation and purification.

A number of methods are useful for the preparation of the salts described above and are known to those skilled in the art. Examples include reaction of the free acid or free base form of the peptide with one or more molar equivalents of the desired acid or base in a solvent or solvent mixture in which the salt is insoluble; or in a solvent like water after which the solvent is removed by evaporation, distillation or freeze drying. Alternatively, the free acid or base form of the product may be passed over an ion-exchange resin to form the desired salt or one salt form of the product may be converted to another using the same general process.

Certain specific schemes that may be appropriate for chemical synthesis of the peptides herein are shown in WO 96/15148 published May 23, 1996, the disclosure of which is incorporated herein by reference.

The peptides of this invention may be administered to the mammal by any suitable technique, including oral, parenteral (*e.g*., intramuscular, intraperitoneal, intravenous, or subcutaneous injection or infusion, or implant), nasal, pulmonary, vaginal, rectal, sublingual, or topical routes of administration, and can be formulated in dosage forms appropriate for each route of administration. The specific route of administration will depend, *e.g*., on the medical history of the patient, including any perceived or anticipated side effects using the compound, the type of peptide being administered, and the particular disorder to be corrected. Optionally, the administration is by continuous infusion (using, *e.g.*, slow-release devices or minipumps such as osmotic pumps or skin patches), or by injection (using, *e.g.*, intravenous or subcutaneous means).

### C. PREPARATION OF TYPICAL FORMULATIONS OF THE INVENTION

In the preparation of typical formulations herein, it is noted that the recommended quality or grade" of the components employed will depend on the ultimate use of the formulation. For therapeutic uses, it is preferred that the component(s) are of an allowable grade (such as "GRAS") as an additive to pharmaceutical products.

In certain embodiments, there are provided compositions comprising Apo-2L receptor binding peptide and one or more excipients which provide sufficient ionic strength to enhance solubility and/or stability of the Apo-2L receptor binding peptide, wherein the composition has a pH of 6 (or about 6) to 9 (or about 9). The Apo-2L receptor binding peptide may be prepared by any suitable method to achieve the desired purity of the protein, for example, according to the above methods. In certain embodiments, the Apo-2L receptor binding peptide is recombinantly expressed in E. coli host cells or prepared by chemical synthesis. The concentration of the Apo-2L receptor binding peptide in the formulation may vary depending, for instance, on the intended use of the formulation. Those skilled in the art can determine without undue experimentation the desired concentration of the Apo-2L receptor binding peptide.

The one or more excipients in the formulations which provide sufficient ionic strength to enhance solubility and/or stability of the Apo-2L receptor binding peptide is optionally a polyionic organic or inorganic acid, aspartate, sodium sulfate, sodium succinate, sodium acetate, sodium chloride, Captisol^{™}, Tris, arginine salt or other amino acids, sugars and polyols such as trehalose and sucrose. Preferably the one or more excipients in the formulations which provide sufficient ionic strength is a salt. Salts which may be employed include but are not limited to sodium salts and arginine salts. The type of salt employed and the concentration of the salt are preferably such that the formulation has a relatively high ionic strength which allows the Apo-2L receptor binding peptide in the formulation to be stable. Optionally, the salt is present in the formulation at a concentration of about 20 mM to about 0.5 M.

The composition preferably has a pH of 6 (or about 6) to 9 (or about 9), more preferably about 6.5 to about 8.5, and even more preferably about 7 to about 7.5. In a preferred aspect of this embodiment, the composition will further comprise a buffer to maintain the pH of the composition at least about 6 to about 8. Examples of buffers which may be employed include but are not limited to Tris, HEPES, and histidine. When employing Tris, the pH may optionally be adjusted to about 7 to 8.5. When employing Hepes or histidine, the pH may optionally be adjusted to about 6.5 to 7. Optionally, the buffer is employed at a concentration of about 5 mM to about 50 mM in the formulation, and preferably at a concentration of about 10 mM to about 20 mM.

Particularly for liquid formulations (or reconstituted lyophilized formulations), it may be desirable to include one or more surfactants in the composition. Such surfactants may, for instance, comprise a non-ionic surfactant like TWEEN^{™} or PLURONICS^{™} (e.g., polysorbate or poloxamer). Preferably, the surfactant comprises polysorbate 20 ("Tween 20"). The surfactant will optionally be employed at a concentration of about 0.005% to about 0.2%.

The formulations of the present invention may include, in addition to Apo-2L receptor binding peptide and those components described above, further various other excipients or components. Optionally, the formulation may contain, for parenteral administration, a pharmaceutically or parenterally acceptable carrier, i.e., one that is non-toxic to recipients at the dosages and concentrations employed and is compatible with other ingredients of the formulation. Optionally, the carrier is a parenteral carrier, such as a solution that is isotonic with the blood of the recipient. Examples of such carrier vehicles include water, saline or a buffered solution such as phosphate-buffered saline (PBS), Ringer's solution, and dextrose solution. Various optional pharmaceutically acceptable carriers, excipients, or stabilizers are described further in Remington's Pharmaceutical Sciences, 16th edition, Osol, A. ed. (1980).

The formulations herein also may contain one or more preservatives. Examples include octadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols, alkyl parabens such as methyl or propyl paraben, and m-cresol. Antioxidants include ascorbic acid and methionine; preservatives (such as octadecyldimethylbenzyl ammonium chloride; hexamethonium chloride; benzalkonium chloride, benzethonium chloride; butyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; sugars such as sucrose, mannitol, trehalose or sorbitol; or polyethylene glycol (PEG).

Additional examples of such carriers include lecithin, serum proteins, such as human serum albumin, buffer substances such as glycine, sorbic acid, potassium sorbate, partial glyceride mixtures of saturated vegetable fatty acids, water, salts, or electrolytes such as protamine sulfate, sodium chloride, polyvinyl pyrrolidone, and cellulose-based substances. Carriers for gel-based forms include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. Conventional depot forms include, for example, microcapsules, nano-capsules, liposomes, plasters, inhalation forms, nose sprays, and sustained-release preparations.

The compositions of the invention may comprise liquid formulations (liquid solutions or liquid suspensions), and lyophilized formulations, as well as suspension formulations in which, the Apo-2L receptor binding peptide is in the form of crystals or amorphous precipitate.

The final formulation, if a liquid, is preferably stored frozen at ≤ 20° C. Alternatively, the formulation can be lyophilized and provided as a powder for reconstitution with water for injection that optionally may be stored at 2-30° C.

The formulation to be used for therapeutic administration must be sterile. Sterility is readily accomplished by filtration through sterile filtration membranes (*e.g.*, 0.2 micron membranes). Therapeutic compositions generally are placed into a container having a sterile access port, for example, an intravenous solution bag or vial having a stopper pierceable by a hypodermic injection needle.

The composition ordinarily will be stored in single unit or multidose containers, for example, sealed ampules or vials, as an aqueous solution or as a lyophilized formulation for reconstitution. The containers may any available containers in the art and filled using conventional methods. Optionally, the formulation may be included in an injection pen device (or a cartridge which fits into a pen device), such as those available in the art (see, e.g., US Patent 5,370,629), which are suitable for therapeutic delivery of the formulation. As an example of a lyophilized formulation, 10 mL vials are filled with 5.5 mL of sterile-filtered 2% (w/v) aqueous Apo-2L receptor binding peptide solution, and the resulting mixture is lyophilized. An injection solution can be prepared by reconstituting the lyophilized Apo-2L receptor binding peptide formulation using, for example, Water-for-Injection.

### D. METHODS OF USE AND OTHER APPLICATIONS

The Apo-2L receptor binding peptides described herein can be employed in methods of treating cancer or immune related conditions. Such therapeutic and non-therapeutic applications are further described, for instance, in WO97/25428, WO97/01633, and WO 01/22987.

The invention contemplates using gene therapy for treating a mammal, using nucleic acid encoding the Apo-2L receptor binding peptide. Nucleic acids which encode the Apo-2L receptor binding peptide can be used for this purpose. Once the amino acid sequence is known, one can generate several nucleic acid molecules using the degeneracy of the genetic code, and select which to use for gene therapy.

There are two major approaches to getting the nucleic acid (optionally contained in a vector) into the patient's cells for purposes of gene therapy: *in vivo* and *ex vivo*. For *in vivo* delivery, the nucleic acid is injected directly into the patient, usually at the site where the Apo-2L receptor binding peptide compound is required. For ex vivo treatment, the patient's cells are removed, the nucleic acid is introduced into these isolated cells and the modified cells are administered to the patient either directly or, for example, encapsulated within porous membranes which are implanted into the patient. See, *e.g.* U.S. Patent Nos. 4,892,538 and 5,283,187.

There are a variety of techniques available for introducing nucleic acids into viable cells. The techniques vary depending upon whether the nucleic acid is transferred into cultured cells *in vitro*, or in vivo in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for *ex vivo* delivery of the gene is a retrovirus.

The currently preferred in vivo nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, *e.g.*, capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu et al., J. Biol. Chem., 262: 4429-4432 (1987); and Wagner et al., Proc. Natl. Acad. Sci. USA, 87: 3410-3414 (1990). For review of the currently known gene marking and gene therapy protocols, see Anderson et al., Science, 256: 808-813 (1992). See also WO 93/25673 and the references cited therein.

In the methods of the invention for treating a disorder, a formulation of Apo-2L receptor binding peptide can be directly administered to the mammal by any suitable technique, including infusion or injection. The specific route of administration will depend, *e.g.*, on the medical history of the patient, including any perceived or anticipated side effects using Apo-2L receptor binding peptide and the particular disorder to be corrected. Examples of parenteral administration include subcutaneous, intramuscular, intravenous, intraarterial, and intraperitoneal administration of the composition. The formulations are preferably administered as repeated intravenous (i.v.), subcutaneous (s.c.), intramuscular (i.m.) injections or infusions, intracranial infusions or as aerosol formulations suitable for intranasal or intrapulmonary delivery (for intrapulmonary delivery see, *e.g.*, EP 257,956).

It is noted that osmotic pressure of injections may be important in subcutaneous and intramuscular injection. Injectable solutions, when hypotonic or hypertonic, may cause pain to a patient upon infusion. Usually, for the therapeutic, injectable formulations herein, it is preferred that the relative osmolarity of the injectable solution be about 300 mosm to about 600 mosm.

Apo-2L receptor binding peptide can also be administered in the form of oral or sustained-release preparations. Suitable examples of sustained-release preparations include semipermeable matrices of solid hydrophobic polymers containing the protein, which matrices are in the form of shaped articles, *e.g*., films, or microcapsules. Examples of sustained-release matrices include cellulose derivatives (*e.g.*, carboxymethylcellulose), sucrose-acetate isobutyrate (SABER^{™}) in non-aqueous media, polyesters, hydrogels (*e.g*., poly(2-hydroxyethyl-methacrylate) (Langer et al., J. Biomed. Mater. Res. 1981, 15: 167-277; Langer, Chem. Tech. 1982, 12: 98-105 or poly(vinylalcohol)), polylactides (U.S. Patent No. 3,773,919, EP 58,481), copolymers of L-glutamic acid and gamma ethyl-L-glutamate (Sidman et al., Biopolymers 1983, 22: 547-556), non-degradable ethylene-vinyl acetate (Langer *et al*., *supra*), degradable lactic acid-glycolic acid copolymers such as the Lupron Depot (injectable microspheres composed of lactic acid-glycolic acid copolymer and leuprolide acetate), and poly-D-(-)-3-hydroxybutyric acid (EP 133,988). One optional method of delivery for systemic-acting drugs involves administration by continuous infusion (using, *e.g*., slow-release devices or minipumps such as osmotic pumps or skin patches), or by injection (using, *e.g.*, intravenous or subcutaneous means, including single-bolus administration).

The composition to be used in the therapy will be formulated and dosed in a fashion consistent with good medical practice, taking into account the clinical condition of the individual patient, the site of delivery of the composition, the method of administration, the scheduling of administration, and other factors known to practitioners. The "effective amounts" of each component for purposes herein are thus determined by such considerations and are amounts that result in bioavailability of the Apo-2L receptor binding peptide or other drugs to the mammal.

It is contemplated that yet additional therapies may be employed in the methods. The one or more other therapies may include but are not limited to, administration of radiation therapy, cytokine(s), growth inhibitory agent(s), chemotherapeutic agent(s), cytotoxic agent(s), tyrosine kinase inhibitors, ras farnesyl transferase inhibitors, angiogenesis inhibitors, and cyclin-dependent kinase inhibitors which are known in the art and defined further with particularity in Section I above, and may be administered in combination (*e.g.*, concurrently or sequentially) with Apo-2L receptor binding peptide(s). In addition, therapies based on therapeutic antibodies that target tumor or other cell antigens such as CD20 antibodies (including Rituxan^{™} ) or Her receptor antibodies (including Herceptin^{™}) as well as anti-angiogenic antibodies such as anti-VEGF, or antibodies that target Apo2L receptors, such as DR5 or DR4.

Preparation and dosing schedules for chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

It may be desirable to also administer antibodies against other antigens, such as antibodies which bind to CD20, CD11a, CD18, CD40, ErbB2, EGFR, ErbB3, ErbB4, vascular endothelial factor (VEGF), or other TNFR family members (such as OPG, TNFR1, TNFR2). Alternatively, or in addition, two or more antibodies binding the same or two or more different antigens disclosed herein may be co-administered to the patient. Sometimes, it may be beneficial to also administer one or more cytokines to the patient.

The Apo-2L receptor binding peptide formulation may be administered in any of the therapeutic methods described in this application in combination with, e.g., concurrently or sequentially, with other agents, cytokines, chemotherapies, antibodies, etc. that are for example, specifically provided in the Definition section of the application above. For example, the Apo-2L receptor binding peptide formulation may be administered as a pre-treatment (prior to administration of any such other agents), such as a pre-treatment of cancer cells which may otherwise be resistant to the apoptotic effects of other therapeutic agents.

As noted above, Apo-2L receptor binding peptides of the invention have various utilities. For example, DR5 agonistic peptides may be employed in methods for treating pathological conditions in mammals such as cancer or immune-related diseases. In the methods, the DR5 binding peptide, preferably an agonistic binding peptide, is administered to a mammal, alone or in combination with still other therapeutic agents or techniques.

Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like. Immune related diseases can also be readily identified. In systemic lupus erythematosus, the central mediator of disease is the production of auto-reactive antibodies to self proteins/tissues and the subsequent generation of immune-mediated inflammation. Multiple organs and systems are affected clinically including kidney, lung, musculoskeletal system, mucocutaneous, eye, central nervous system, cardiovascular system, gastrointestinal tract, bone marrow and blood.

Medical practitioners are familiar with a number diseases in which intervention of the immune and/or inflammatory response have benefit. For example, rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, interstitial pneumonitis with pulmonary fibrosis, keratoconjunctivitis sicca, and rheumatoid nodules.

Juvenile chronic arthritis is a chronic idiopathic inflammatory disease which begins often at less than 16 years of age. Its phenotype has some similarities to RA; some patients which are rheumatoid factor positive are classified as juvenile rheumatoid arthritis. The disease is subclassified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing spondylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-827 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjogren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barr syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are Infectious disease including but not limited to viral infection (including but not limited to AIDS, hepatitis A, B, C, D, E) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (i.e. as from chemotherapy) immunodeficiency), and neoplasia.

Also provided is a method for predicting the relative affinity for binding to a ligand of a peptide that competes with a polypeptide for binding to the ligand, which peptide is derived from a phage-displayed library, which method comprises incubating a phagemid clone corresponding to the peptide with the polypeptide in the presence of the ligand, serially diluting the phage, and measuring the degree to which binding of the phagemid clone to the ligand is inhibited by the peptide, wherein a phagemid clone that is inhibited only at low phage concentrations has a higher affinity for the ligand than a phagemid clone that is inhibited at both high and low phage concentrations.

Diagnostic methods are also provided herein. For instance, the Apo2L receptor binding peptides may be employed to detect the respective DR4 or DR5 receptors in mammals known to be or suspected of having a Apo-2L, DR4 or DR5 related pathological condition. The binding peptides may be used, e.g., in assays to detect or quantitate DR4 or DR5 in a sample. A sample, such as cells obtained from a mammal, can be incubated in the presence of a labeled binding peptide, and detection of the labeled binding peptide bound in the sample can be performed. Such assays, including various clinical assay procedures, are known in the art, for instance as described in Voller et al., Immunoassays, University Park, 1981.

The invention also provides kits which include Apo-2L receptor binding peptides described herein. A typical kit will comprise a container, preferably a vial, for Apo-2L receptor binding peptide in one or more excipients as described above; and instructions, such as a product insert or label, directing the user as to how to employ the Apo-2L receptor binding peptide formulation. This would preferably provide a pharmaceutical formulation. Preferably, the pharmaceutical formulation is for treating cancer or an immune related condition. Suitable containers include, for example, bottles, vials, syringes, and test tubes. The containers may be formed from a variety of materials such as glass or plastic. The container holds an Apo-2L receptor binding peptide formulation that is effective for diagnosing or treating the disorder and may have a sterile access port (for example, the container may be an intravenous solution bag or a vial having a stopper pierceable by a hypodermic injection needle). The label on, or associated with, the container indicates that the formulation is used for diagnosing or treating the disorder of choice. The article of manufacture may further comprise a second container comprising water-for-injection, a pharmaceutically-acceptable solution, saline, Ringer's solution, or dextrose solution. It may further include other materials desirable from a commercial and user standpoint, including other buffers, diluents, filters, needles, syringes, and package inserts with instructions for use.

### EXAMPLES

The following examples are offered for illustrative purposes only, and are not intended to limit the scope of the present invention in any way. Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1: Phage-derived Peptides Bind to Apo-2L receptors.

In the next set of examples, common α-amino acids may be described by the standard one- or three-letter amino acid code when referring to intermediates and final products. By common α-amino acids is meant those amino acids incorporated into proteins under mRNA direction. Standard abbreviations are listed in The Merck Index, 10th Edition, pp Misc-2 - Misc-3. Unless otherwise designated the common α-amino acids have the natural or "L"- configuration at the alpha carbon atom. If the code is preceded by a "D" this signifies the opposite enantiomer of the common α-amino acid. Modified or unusual α-amino acids such as norleucine (Nle) and ornithine (Orn) are designated as described in U.S. Patent and Trademark Office Official Gazette 1114 TMOG, May 15, 1990.

It has been shown that peptides which bind specifically and with measurable affinity to target molecules, such as proteins, can be identified from an initial library of many binding and non-binding peptides through binding selections using bacteriophage coat-protein fusions. Smith, Science, 228: 1315 (1985); Scott and Smith, Science, 249: 386 (1990); Cwirla et al., Proc. Natl. Acad. Sci. USA, 8: 309 (1990); Devlin et al., Science, 249: 404 (1990); reviewed by Wells and Lowman, Curr. Opin. Struct. Biol., 2: 597 (1992); U.S. Pat. No. 5,223,409. In addition, both proteins and peptides displayed on phage can be affinity-enhanced through iterative cycles of mutations, selection, and propagation.

Libraries of peptides differing in sequence at particular residue positions can be constructed using synthetic oligodeoxynucleotides. Peptides are displayed as fusion proteins with a phage coat protein (such as g3p or g8p) on bacteriophage particles, each of which contains a single-stranded DNA genome encoding the particular peptide variant. After cycles of affinity purification, using an immobilized target molecule, individual bacteriophage clones are isolated, and the amino acid sequence of their displayed peptides is deduced from their DNA sequences.

### I. Construction of peptide-phage libraries

To identify peptide molecules having the ability to bind to an Apo-2L receptor such as DR4 or DR5, several diverse phage libraries of peptides, of length ranging from about 12 to about 24 amino acid residues, were constructed. Peptides of this length or size were chosen in part to tend to favor the selection of peptides capable of maintaining well-defined structures in solution. Natural-amino acid peptides of this size have a potential sequence diversity of 20¹⁸-20²⁰ (*i.e.*, 2.6 x 10²³ to 1.0 x 10²⁶) variants. Certain residues were fixed or constant, which might be expected to allow or promote stable elements of peptide structure such as disulfide bonds or beta-turns, within each peptide.

Used as a template for library constructions was a plasmid, pt4.g8 expressing an antibody-recognizable (gD-tag) peptide fused to g8p of bacteriophage M13. This plasmid contains single-stranded and doublestranded origins of DNA replication. The *phoA* promoter and STII secretion-signal sequences are upstream of the gD peptide (underlined below), which is followed by a "linker" peptide (double underlined below), and then the g8p of bacteriophage M13:

SGTAMADPNRFRGKDLAGSPGGGSGGGAEGDDPAKAAFNSLQASATEYIGYAWAMVVVIVGATIGIKLFKKFTSK
AS (SEQ ID NO: 7)

Several random-sequence peptide libraries were constructed using single-stranded template-directed mutagenesis (Kunkel et al., Methods. Enzymol., 204:125 (1991)). Tables I and II below identify these libraries.

The Apo-2L receptor binding peptides disclosed herein were identified from the bacteriophage peptide library constructs shown in Tables I and II using the following phagemid binding assay.

### PHAGEMID BINDING ASSAY

1. Coat microtiter plate wells with 80ul of 1ug/ml Apo-2L receptor DR5 ECD (SEQ ID NO: 105) (in 50mM sodium carbonate pH 9.6) at 4° C overnight.
2. Remove coat and block with 200ul PBS/0.1% BSA. Incubate 1 hour at room temperature.
3. Prepare competing receptor solutions (100ul/sample).
   - Dispense 50ul binding buffer to row H through B in a new non-sticky 96-well plate.
   - Dispense 75ul competing receptor solution to row A.
   - Make 3-fold serial dilutions by transferring 25ul solution along columns except for row H (use fresh tip set for each transfer).
4. Prepare appropriate subsaturating phage dilution (predetermined from phage dilution series).
   - Transfer 50ul of phage diluent to each microtiter well of the non-sticky plate. Start at row H then G,F,E,D,C,B,A respectively.
   - Incubate at R.T. for 2 hours.
5. Shake block off plate which has been coated with DR5 ECD (SEQ ID NO: 105) and rinse 10 times with PBS + 0.05% tween 20.
6. Transfer 80ul the mixture of competing receptor and phage from the non-sticky plate to DR5 ECD coated plate and incubate 20 minutes at room temperature.
7. Make a 1:5000 dilution of HRP conjugate anti-M13 monoclonal Ab (Amersham Pharmacia Biotech) into binding buffer.
   - Rinse plate 10 times with PBS/tween20 (be thorough here to remove vast excess of non displaying phage).
   - Add 80ul of Ab diluent to row H through A.
   - Incubate 1 hour at R.T.
8. Mix TMB peroxidase substrate and peroxidase solution B at equal volume.
   - Rinse plate 10 times with PBS/tween20.
   - Add 80ul of substrate to row H through A.
   - Incubate as required to develop and stop with 80ul 2.5M H₂SO₄.

Apo-2L receptor binding peptides identified using this assay were then affinity sorted using the following protocols.

### AFFINITY SORTING -PEPTIDE METHOD 1:

### Sort 1

1. Coat target protein (human DR5 ECD, SEQ ID NO: 105) on Maxisorp immunoplate 24 wells with 2ug/ml, 200ul/well, and incubate at 4°C overnight.
2. Block the plate: Add 200ul of PBS with casein for 1 hour at room temperature.
3. Block phage: Add blocking buffer (casein) to the phage solution at 1:1 ratio and incubate at room temperature for 1 hour.
4. Wash the plate 5 times with PT buffer.
5. Add 100uL of library phage solution (from step 4) to the wells and incubate at room temperature for 2 hrs. with gentle shaking.
6. Wash the plate 10 times with PT buffer.
7. Elute with 50ul of 0.2 M glycine, pH 2 at the first 12 wells for 30 mins., then transfer the solution to the second 12 wells and incubate for 30 mins. Neutralize the eluant with 1.0M tris base (about 1/6 volume).
8. Infect 5 ml of X11-blue cells (0D600=1.0) with 1.5 ml phage eluant. Grow 20 mins. At 37°C. Titer on LB/carb plates and transfer the culture to 50mL of 2YT/carbVCS and grow overnight at 37°C.
9. Spin down the cells and save the supernatant which is ready for the next sort.

### Sort 2

1. Coat 12 wells with target protein.
2. Block the plate: Add 200ul super block for 1 hr. at room temperature.
3. Block the phage: Add superblock (Pierce, CA) to phage supernatant (from sort 1 step 12) at ratio 1:1 and incubate at room temperature for 1 hr.
4. Infect 1 ml of X11-blue cells (OD600=1.0) with 0.3 ml phage eluant. Grow 20 mins. at 37°C. Titer on LB/carb plates and transfer the culture to 25mL of 2YT/carbvCs and grow overnight at 37°C.
5. Spin down the cells and save the supernatant which is ready for the next sort.

### Sort 3

1. Coat 12 wells with target protein.
2. Block the plate: Add 200ul PBS/BSA for 1 hr. at room temperature.
3. Block the phage: Add *PBS*/*PBS* to phage supernatant at ratio 1:1 and incubate at room temperature for 1 hr.
4-5. The same as above.

### AFFINITY SORTING -PEPTIDE METHOD 2

### Sort 1

1. Block beads.
4. Mix the beads thoroughly then remove 600ul beads to eppendorf tube.
5. Wash 3 times with 1 ml PBS / BSA.
6. Resuspend washed beads in PBS / BSA.
7. Place on rotator at room temperature for 1 hr.
2. Block phage:
   a) Add blocking buffer (PBS / BSA) to peptide phage library at volume ratio 1:1.
   b) Place on rotator at room temperature for 1/2 hr.
   c) Add 50nM Dr4 IgG (SEQ ID NO: 108) (for Dr5-specific) or Dr5 IgG (SEQ ID NO: 109)(for Dr4-specific).
   d) Place on rotator at room temperature for 1 hr.
3. Bind peptide phage to antigen:
   a) Add 100nM biotin-labeled target protein to the phage solution.
   b) Rotate at room temperature for 1-2 hrs.
4. Transfer the phage-mixture (from step 3) to the blocked-beads (from step1) and place on the rotator for 15 mins.
5. Wash the beads with PBS / Tween 10 times.
6. Elute the phage with 1OOuL 0.2M glycine, pH 2 for 30 mins, then neutralize the eluant with 1.0M tris base (about 1/6 volume).
7. Infect 5 ml of X11-blue cells (OD600=1.0) with 0.5 ml phage eluant. Grow 20 mins. at 37°C. Titer on LB/carb plates and transfer the culture to 25mL of 2YT/carbVCS and grow overnight at 37°C.
8. Spin down the cells and save the supernatant which is ready for the next sort.

### Sort 2

Similar as sort 1, Concentration: see chart A below. Block solution with Casein.

### Sort 3

Similar as sort 1, Concentration: see chart A below. Block solution with super block.

### Sort 4

If desired. Similar as sort 1, Concentration: see chart A below. Block with 5% skim milk.

### CHART A

| Round | Beads (ml) | Biotin-Antigen (nM) (DR5-IgG) | Competitive-protein (nM) (DR4-IgG) |
|---|---|---|---|
| | | (SEQ ID NO: 109) | (SEQ ID NO: 108) |
| Sort 1 | 0.6 | 100 | 50 |
| Sort 2 | 0.3 | 100 | 100 |
| Sort 3 | 0.3 | 10 | 200 |
| Sort 4 | 0.3 | 1 | 200 |

Several screening protocol strategies were employed to identify and isolate the peptides disclosed herein. A first protocol consisted of an early and mass ("E.M.") screening strategy in which clones were selected after two or three sorting steps. In this E.M. strategy, early and mass selection methods were employed to identify peptide sequences having a relative diversity of binding epitopes (e.g. diversity in both sequence structure and binding affinity).

A second protocol employed to identify the peptides disclosed herein consisted of a late and limited ("L.L.") screening strategy in which clones were selected after additional (e.g. four to six) sorting steps. The L.L. screening strategy was used to select for higher affinity peptide species.

The materials and methods used in these peptide screening protocols are as follows:

### A. Materials:

1. Solid Support, NUNC 384-well plate (Cat #781061).
2. Coating Buffer (CB), 0.05 M carbonate/bicarbonate pH 9.6.
3. Wash Buffer (WB), PBS/0.05% Tween-20, pH 7.4.
4. ELISA Diluent w/o Thimerosal (AB), PBS/0.5 % BSA, .05%Tween-20.
5. Substrate (S), TMB.
6. Stop Solution (SS), 1 M phosphoric acid (67.5 mL H₃PO₄ + 932.5 mL dH₂O.
7. Coat target, 2 microgram/mL.
8. Coat anti-gD (5 microgram/mL).
9. Conjugate, anti-M13/HRP 1:5000 dilution (Amersham Pharmacia biotech).

### B. Procedure:

1. a) Grow the phage to 0.4ml of 2YT/cabVCS overnight at 37°C.
   b)Dilute target protein (DR5 ECD, SEQ ID NO: 105) to 2 µg/mL. Add 40 µL to each well. Incubate overnight at 2-8°C.
2. Wash plate on plate washer. (100 µL x 3 washes). Blot dry.
3. Add 100 µL of ELISA Assay Buffer to each well to block the plate. Incubate for 1h at RT with gentle agitation.
4. wash plate on plate washer. (100 µL x 3 washes). Blot dry.
5. Add 30µL sample (Phage-dilutes samples 1/3) to each well. Samples are transferred form a 96 well plate to a 384 plate in quadrants. Incubate for 55 min to 1hr 15 min at room temperature with gentle agitation.
6. Wash plate on plate washer. (100 µL x 6 washes). Blot dry.
7. Add 40 µL of anti-M13/HRP (1:50K) diluted in Assay Buffer to each well. Incubate for 55 min to 1hr 15 min at room temperature with gentle agitation.
8. Wash plate on plate washer(100 µL x 6 washes). Blot dry.
9. Combine equal volumes of substrate and peroxidase solution. Add 40 µL of solution to each well. Allow to incubate until sufficient color develops (-18 to 23 min).
10. Add 40 µL of Stop Solution to each well.
11. Read absorbance at 450 nm and 620 nm for reference.

In the screening process used to identify peptides disclosed herein, steps 4-11 above were carried out by a CRS robotic system (Kernel procedure = Phage).

### PHAGE CLONE PURIFICATION

A number of bacteriophage clones expressing peptides of interest were generated using the above methods and then purified using limiting dilution. In order to further characterize specific clones of interest (e.g. to determine Ic 50 values), phage clones were grown and then purified using a polyethylene glycol based method as described in Sidhu et al., Methods Enzymol. 328, 333-363 (2000). Alternative methods for generating and purifying bacteriophages are also known in the art. See, e.g. Current Protocols In Molecular Biology, Volume 1, Units 1, 5 and 6, Frederick M. Ausubel et al. eds., 1995.

Phage clones identified using the above methods were grown and then purified using the following polyethylene glycol based method. Briefly, a bacterial culture infected with a phage clone of interest is grown under conditions favorable to phage growth and then centrifuged for 10 min at 2°C and 16,000g. The supernatant is then transferred to a fresh centrifuge tube and a 1/5 volume of PEG-NaCl (PEG 8000 (200 g/L), NaCl (146 g/L), autoclaved) is added. The mixture is then incubated for 5 minutes at room temperature. The mixture is then centrifuged as above and the supernatant decanted. The supernatant is then respun briefly and the remaining supernatant removed. The phage pellet is then resuspended in 1/20 volume of PBS (or other analogous buffer). Insoluble matter is then pelleted by centrifugation. The supernatant containing the purified phage is saved and used in assays such as Ic 50 and Ec 50 assays that are described in the following examples.

Peptides of the invention and their Ic 50 values (e.g. determinations of Ic 50 in ELISA based assays using a DR5 ECD (SEQ ID NO: 105) competitor) are identified in Table III below. In the Tables provided herein, "Ic 50" refers to the measure of the concentration of peptide needed to inhibit 50% of Apo-2L (e.g. amino acids 114 to 281 of SEQ ID NO: 1) binding to an Apo-2L receptor molecule (e.g. DR4-IgG (SEQ ID NO: 108) or DR5 ECD (SEQ ID NO: 105) etc.). The peptides are classified such that class 1-class 4 represent a, b, c, and d respectively.

Table IV below provides certain properties of selected peptides, for example Ic50 cross-reactivity data with DR4 and DR5 polypeptides. Table VIII identifies the sequence identification numbers (SEQ ID NOs.) for the various peptides disclosed herein.

### Example 2: Protocols Used to Manipulate and/or Modify Apo-2L Receptor Binding Peptides.

As disclosed in this example, peptides identified using the above methods were modified according to a number of art accepted protocols.

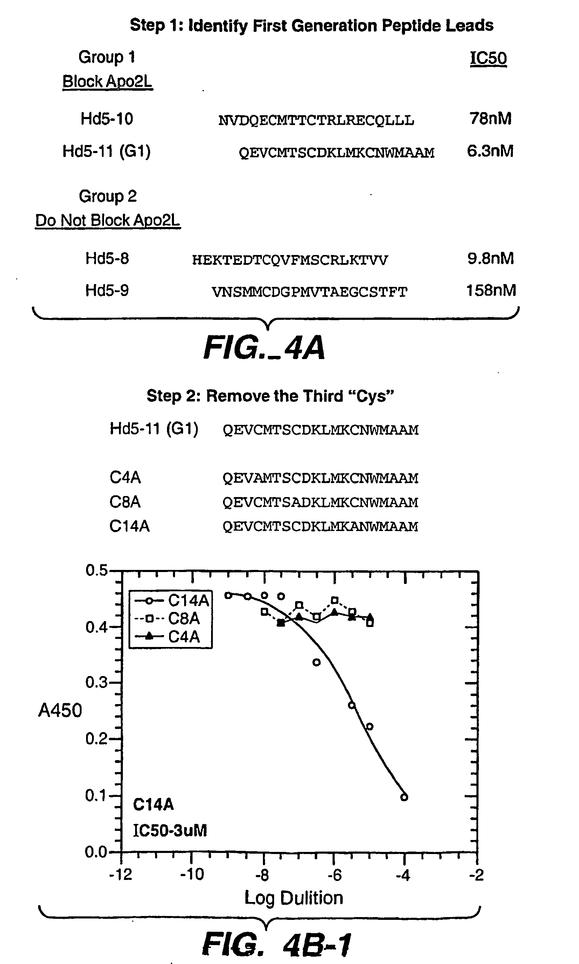

In a first step, specific peptides were selected for further modification. Peptides were selected for such modification based on criteria including an ability to bind to DR5 polypeptides (SEQ ID NOS: 105 and 109) with a specific affinity and/or an ability to inhibit the interaction between Apo-2L.0 (amino acids 114 to 281 of SEQ ID NO: 1) and DR5 (SEQ ID NOs: 105 and 109) at a specific concentration. As shown in Figure 4A, peptides Hd5-10 (SEQ ID NO: 14), Hd5-11 (SEQ ID NO: 15), Hd5-8 (SEQ ID NO: 92) and Hd5-9 (SEQ ID NO: 99) were identified as first generation (G1) peptide leads.

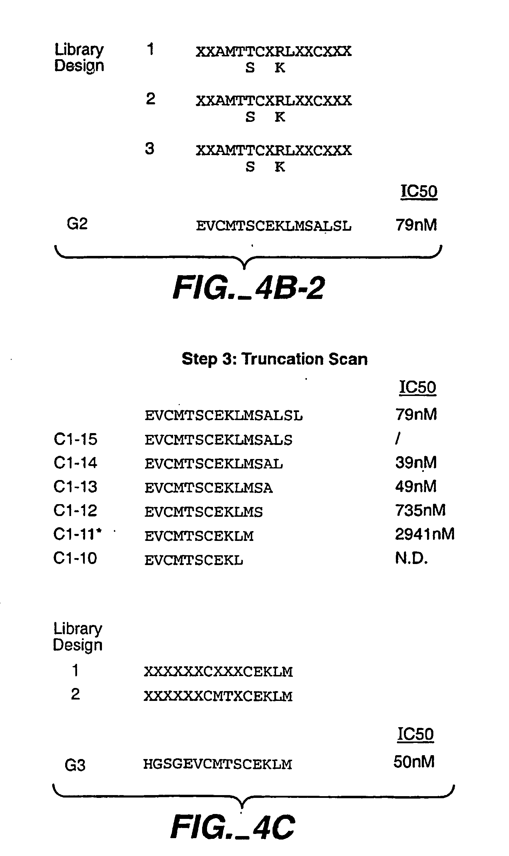

As shown in Figure 4B, original amino acid residues within the Hd5-11 peptide were modified by amino acid substitution. Specific residues within this peptide were selected for modification based on their appearance at a high frequency at the same sequence position within peptides selected as above. As shown in Figure 4B, cysteine residues 4, 8 and 14 within the Hd5-11 peptide sequence were substituted with alanine residues. As shown in the graph in Figure 4B, the effects of the C → A substitution on peptide Ic 50 values were then measured. A library design as shown in Figure 4B facilitated this peptide modification. A second generation (G2) peptide produced by these methods is shown in Figure 4B.

In addition to frequency of occurrence, specific residues within a peptide can be selected for such modification based on a variety of additional criteria including a desire to remove of residues associated with the formation of disulfide bonds (i.e. cysteine) as well as the addition of residues that are particularly amenable to subsequent manipulation (e.g. residues that facilitate conjugation to polyols).

As shown in Figure 4C, the G2 peptide identified in Figure 4B was modified using truncation scanning protocols wherein one or more terminal residues of a peptide are deleted (see, e.g. Bing Li et al., Science 270, 1657 (1995). As shown in the graph in Figure 4C, the effects of truncation on peptide Ic 50 values were measured. A library design as shown in Figure 4C facilitated this peptide modification. A third generation (G3) peptide produced by these methods is shown in Figure 4C.

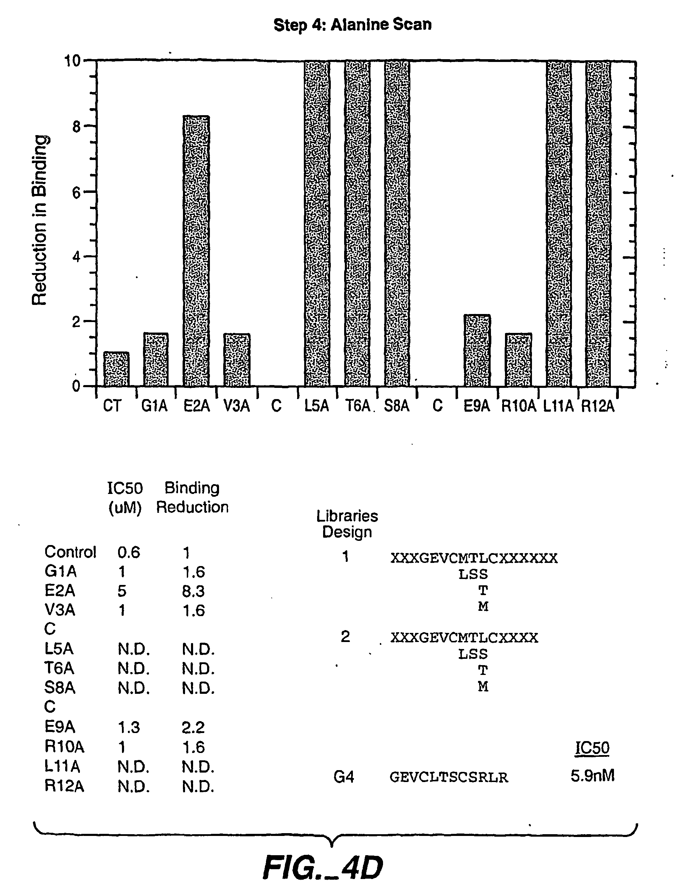

As shown in Figure 4C, amino acid residues within the G3 peptide were modified using alanine scanning protocols (see, e.g. Cunningham B.C. et al. Science 244, 1081 (1989)). In these protocols one or more residues of the G3 peptide were substituted with alanine and the effects of the various substitutions on peptide Ic 50 values was measured. A library design as shown in Figure 4D facilitated this peptide modification. A fourth generation (G4) peptide produced by these methods is shown in Figure 4C.

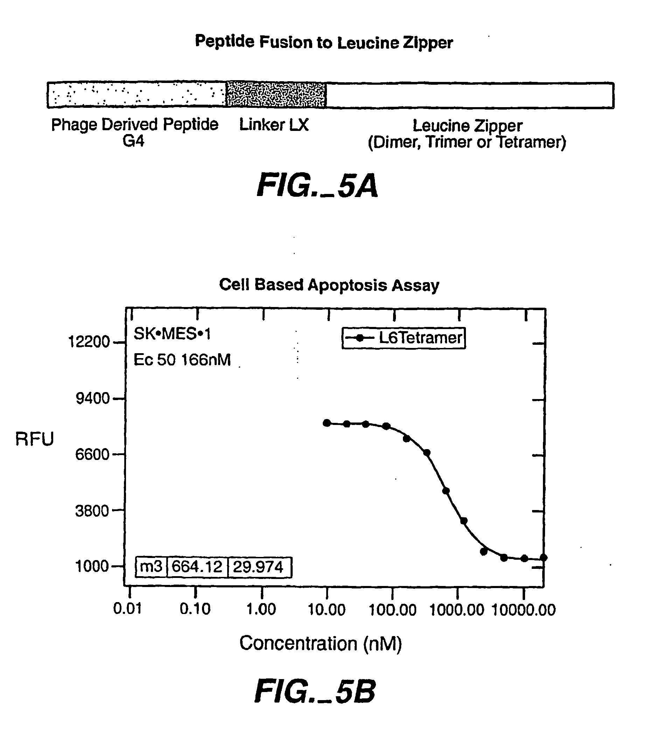

As discussed in Examples 5 below, the effects of peptide modification were examined in assays which evaluated the ability of a peptide to inhibit the interaction between Apo-2L and an Apo-2L receptor. As discussed in Example 4 below (and shown in Figure 5B), the effects of peptide modification were also examined in an assay of the ability of an LB881 Apo-2L receptor binding peptide (SEQ ID NO: 45) that is linked to a leucine zipper sequence to induce apoptosis in SK-MES-1 cells.

### EXAMPLE 3: DR5 binding peptide Oligomerization.

Defined oligomers of the LB881 peptide shown in Table III (SEQ ID NO: 45) were engineered by fusion to the amino terminus of the GCN4 coiled coil (see, e.g. O'Shea et al., (1989) Science 243, 538-542). Trimer and tetramer variants of the GCN4 dimer were based on the sequences reported by Harbury et al. (see, e.g. Harbury et al., (1993) Science 262, 1401-1407) but differed at the ends of the GCN4 sequence. All three GCN4 variants had two additional residues at the amino terminus (Lys and the appropriate hydrophobic residue; see Table V below), and all three ended in the sequence "VGER". The peptides were fused to the GCN4 variants through linkers of different lengths (see Table VI below).

### Methodology:

Expression plasmids for peptide-GCN4 fusions were produced by insertion of a peptide-linker cassette into the NdeI site of GCN4-pR (see, e.g. Cochran, A. G. &; Kim, P. S. (1996) Science 271, 1113-1116) (encoding the GCN4 dimerization domain or the appropriate variant) using standard molecular cloning techniques. Linker variants were then produced by Kunkel mutagenesis of the initial constructs. Plasmids encoding the fusion peptides were transformed into E. coli strain BL21-DE3 pLysS (Novagen). A culture in L broth (LB) was inoculated with a single colony and shaken overnight at 37 °C. An expression culture (1 L in LB) was then inoculated with 10 mL overnight culture and shaken at 37 °C. When the culture reached an absorbance at 550 nm of 0.5, peptide expression was induced with isopropyl-β-D-thiogalactopyranoside (0.4 mM final concentration). After three hours of additional growth, cells were harvested by centrifugation.

Cell pellets were frozen at -20 °C, thawed, and lysed with glacial acetic acid (10 mL per L original culture) at room temperature. Precipitates were removed by centrifugation, and supernatant was loaded onto a 5 cm x 25 cm size exclusion column (Sephadex G75 fine; Pharmacia). The fusion peptide was eluted with 5% (v/v) aqueous acetic acid at a rate of 3 mL min⁻¹. Fractions containing the fusion peptide were identified by liquid chromatograph mass spectrometry (PE SCIEX API 150 EX mass spectrometer with Shimadzu LC-10AD pumps, a Shimadzu dual-wavelength UV detector Model SPD-10AVP, and a Perkin Elmer Series 200 autosampler) using a Microsorb C18 column (part no. R0089203C5, Varian Chromatography Systems) and a gradient of acetonitrile in water (0.05 % (v/v) trifluoroacetic acid). Fusion peptide fractions were combined and lyophilized.

The lyophilized fusion peptide was dissolved in water containing 0.1% trifluoroacetic acid and further purified by reversed-phase preparative HPLC, using a gradient of acetonitrile in water (0.1 % (v/v) trifluoroacetic acid). HPLC fractions containing the purified fusion peptide were combined, oxidized by dropwise addition of iodine-saturated acetic acid, and lyophilized. To decrease the possibility of intermolecular crosslinking during the oxidation process, the lyophilized product was weighed and resuspended under denaturing conditions in 6 M Guanidine HCl, 50 mM Tris, pH 7.5, 1 mM EDTA (buffer A), at a concentration of 2 mg/ml. The denatured peptide was oxidized with aqueous potassium ferricyanide. Monomeric peptide was separated from cross-linked aggregates by gel filtration chromatography on a Pharmacia HiPrep 26/60 Sephacryl S-100 High Resolution column. The running buffer was buffer A. The fractions containing monomeric peptide were dialyzed against 50 mM Tris, pH 7.5, 1 mM EDTA for at least 3 hours at 4° C. Dialyzed fractions were analyzed for purity by polyacrylamide gel electrophoresis. Pure fractions were combined, and the pooled, dialyzed fractions were again purified by reversed-phase HPLC for a final yield of 2-10 mg.

Lyophilized powders were dissolved in water at 25 mg mL⁻¹, and peptide concentrations were determined from absorbance as described for example in Gill, S. C. & von Hippel, P. H. (1989) Calculation of protein extinction coefficients from amino acid sequence data. Anal. Biochem. 182, 319-326. Stock solutions were prepared for assay by dilution of peptides to 2 mM and addition of Na₂HSO₄ to neutralize solution pH.

Table VII below provides data for the LB881 peptide (SEQ ID NO: 45) fused to the amino terminus of the GCN4 coiled coil.

### EXAMPLE 4: Alamar blue Assay of Ability of Apo-2 Receptor binding Peptides to Agonistically induce Apoptosis.

A bioassay which measures cell viability from the metabolic conversion of a fluorescent dye was used to determine the apoptotic activity of the LB881 Apo2L receptor binding peptide shown in Table IX fused to the amino terminus of the GCN4 coiled coil (SEQ ID NO: 113). Data from this bioassay is shown in Figure 5B.

Briefly, serial 2-fold dilutions of Apo-2L.0 or this Apo2L receptor binding peptide were made in RPMI-1640 media (Gibco) containing 0.1% BSA, and 50 µL of each dilution was transferred to individual wells of 96-well Falcon tissue culture microplates. The terms "Apo-2L.0" and "Apo2L.0" refer to a form of Apo-2 ligand polypeptide consisting of amino acids 114 to 281 of Figure 1 (amino acids 114 to 281 of SEQ ID NO: 1) and not linked or conjugated to any epitope tag sequences. After the Apo-2L.0 or Apo2L receptor binding peptide dilution step, 50 µL of SK-MES-1 human lung carcinoma cells (ATCC HTB58) (in RMPI-1640, 0.1% BSA) were added at a density of 2 x 10⁴ cells/well. These mixtures were incubated at 37°C for 24 hours. At 21 hours, 25 µL of alamarBlue (AccuMed, Inc., Westlake, Ohio) was added. Fluorescence was read using 96-well fluorometer with excitation at 530 nm and emission of 590 nm. The results are expressed in relative fluorescence units (RFU). For data analysis the 4-parameter curve fitting program (Kaleidagraph) was used.

Using these bioassay methods, the Ec 50 values for Apo-2L LB881 receptor binding peptides were generated and are provided in the Table VII below. "Ec 50" refers to the median effective concentration of peptide observed to induce apoptosis in 50% of the cell population.

### EXAMPLE 5: Assay for Apo-2 Receptor binding Peptides Ability to Inhibit Binding of Apo-2L to Apo-2L Receptor.

Purified Apo-2L receptor binding peptides of the invention (as described in Example 1 above) were tested for activity to inhibit binding of Apo-2 ligand (amino acids 114 to 281 of SEQ ID NO: 1) to the Apo-2L receptor construct human DR5-ECD (SEQ ID NO: 105). Briefly, in the ELISA, 96-well microtiter plates (Maxisorp; Nunc, Kamstrup, Denmark) were coated by adding 100 µl of 5µg/ml human DR5-ECD (SEQ ID NO: 105) in Coating Buffer (50 mM Carbonate buffer pH 9.6) to each well and incubating at 4°C overnight. The plates were then washed three times with washing buffer (PBS containing 0.05% Tween 20). The wells were then blocked with 200 µl of 2% bovine serum albumin in PBS and incubated at room temperature for 1 hour. The plates were then washed again three times with wash buffer.

Following the wash steps, 100 µl of a three-fold serial dilution of purified Apo-2 Receptor binding Peptide (50 µg/ml) in assay buffer (0.5% bovine serum albumin, 0.05% Tween 20 in PBS) was added to designated wells. The plates were then incubated at room temperature for 1 hour on a shaker apparatus and washed three times with wash buffer.

Next, 100 µl of Biotinylated Apo-2L.0 (amino acids 114 to 281 of SEQ ID NO: 1) diluted 1:1000 in assay buffer was added to each well and the plates incubated for 1 hour at room temperature on a shaker. The plates were then washed three times with wash buffer followed by the addition of 100 µl of Streptavidin-HRP (Zymed, South San Francisco CA) diluted 1:1000 in assay buffer and incubated 1 hour at room temperature on a shaker apparatus and then washed three times with wash buffer.

Followed by addition of 50 µl of substrate (TMB Microwell peroxidase substrate, Kirkegaard & Perry, Gaithersburg, MD) to each well and incubated at room temperature for 10 minutes. 50 µl of TMB-1 component stop solution (Diethyl Glycol; Kirkegaard & Perry, Gaithersburg, MD) was added to each well to stop the reaction, and absorbance at 450 nm was read in an automated microtiter plate reader.

Using these methods, the Ic 50 values for various phage clones expressing peptide sequences of interest were generated and are provided in the Tables below. In the Tables, "Ic 50" refers to the measure of the concentration of peptide needed to inhibit 50% of Apo-2L binding to an Apo-2L receptor molecule (e.g. DR4-IgG (SEQ ID NO: 108) or DR5 ECD (SEQ ID NO: 105) etc.).

While this example describes an ELISA assay protocol which uses a DR5-ECD construct (SEQ ID NO: 105) as a target receptor molecule, the ability these Apo-2L receptor binding peptides to bind other Apo-2L receptor molecules can be examined using these ELISA based methods.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

### TABLES

### TABLES I-II: NAIVE LIBRARIES FOR G8 DISPLAY

**Table I: G1-G2.**

| Library code | | Design | Diversity (X10¹⁰) |
|---|---|---|---|
| Class 1 | | | |
| G1-1 | (g8) | C-X2-GP-X4-C | 2.5 |
| G1-2 | (g8) | X4-C-X2-GP-X4-C-X4 | 2.0 |
| G1-3 | (g8) | X20 | 1.2 |
| G1-4 | (g8) | X7-C-X4-C-X7 | 2.5 |
| G1-5 | (g8) | X7-C-X5-C-X6 | 1.4 |
| G1-6 | (g8) | X6-C-X6-C-X6 | 2.5 |
| G1-7 | (g8) | X6-C-X7-C-X5 | 2.1 |
| G1-8 | (g8) | X5-C-X8-C-X5 | 1.9 |
| G1-9 | (g8) | X5-C-X9-C-X4 | 2.0 |
| G1-10 | (g8) | X4-C-X10-C-X4 | 2.5 |
| G1-11 | (g8) | X8 | 2.6 |
| G1-12 | (g8) | X2-C-X4-C-X2 | 2.1 |
| G1-13 | (g8) | X2-C-X5-C-X2 | 2.2 |
| G1-14 | (g8) | X2-C-X6-C-X2 | 1.5 |
| G1-15 | (g8) | X2-C-X7-C-X2 | 2.1 |
| G1-16 | (g8) | X2-C-X8-C-X2 | 2.1 |
| G1-17 | (g8) | X2-C-X9-C-X2 | 2.2 |
| G1-18 | (g8) | X2-C-X10-C-X2 | 2.4 |
| | | | |
| G2-836 | (g8) | XXCMTT(or S)CXR(or K) LXXCXXX | 0.9 |
| G2-837 | (g8) | XXAMTT(or S)CXR(or K) LXXCXXX | 2.0 |
| G2-838 | (g8) | XXCMTT(or S)AXR(or K)LXXCXXX | 1.0 |
| G2-839 | (g8) | XXCMTT(or S)CXR(or K)LXXAXXX | 1.0 |
| | | | |

**Table II: G3-G5.**

| Library code | | Design | Diversity (X10 ¹⁰) |
|---|---|---|---|
| Class 1 | | | |
| G3-857 | (g8) | XXXXXXCXXXCEKLM | 2.0 |
| G3-858 | (g8) | XXXXXXCMTXCEKLM | 0.9 |
| | | | |
| G4-870 | (g3) | XXXEGVCM(L) T(S) L(S/T/M)CXXXXXX | 1.0 |
| G4-871 | (g3) | XXXEGVCM(L) T(S) L(S/T/M)CXXXX | 1.0 |
| | | | |
| G5-927 | (g3) | XEXCLTSCXXLR | 2.0 |
| G5-928 | (g3) | XXXECLTSCXXLRXX | 2.0 |
| G5-929 | (g3) | GXVCXXXCSRXX | 0.8 |
| G5-930 | (g3) | XXGXVCXXXCSRXXXX | 0.9 |
| | | | |

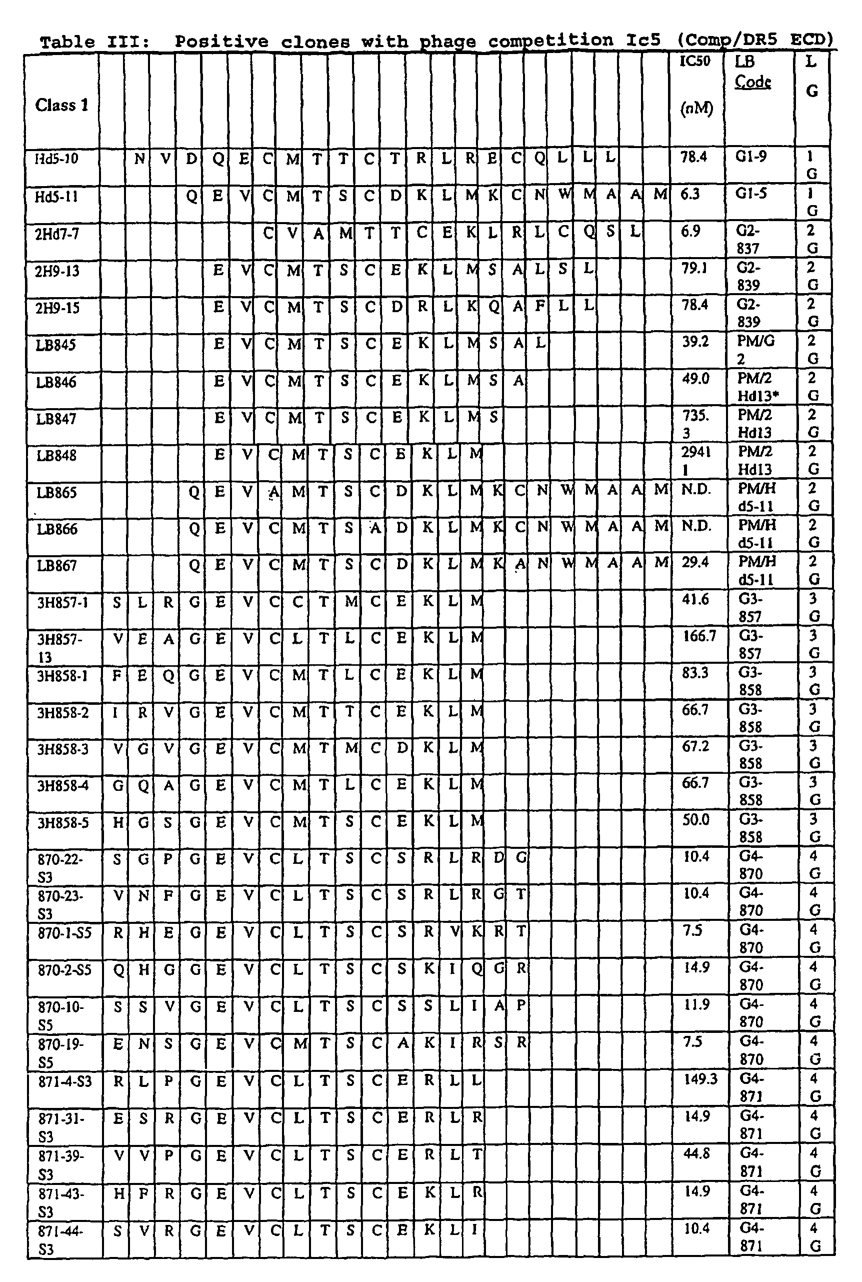

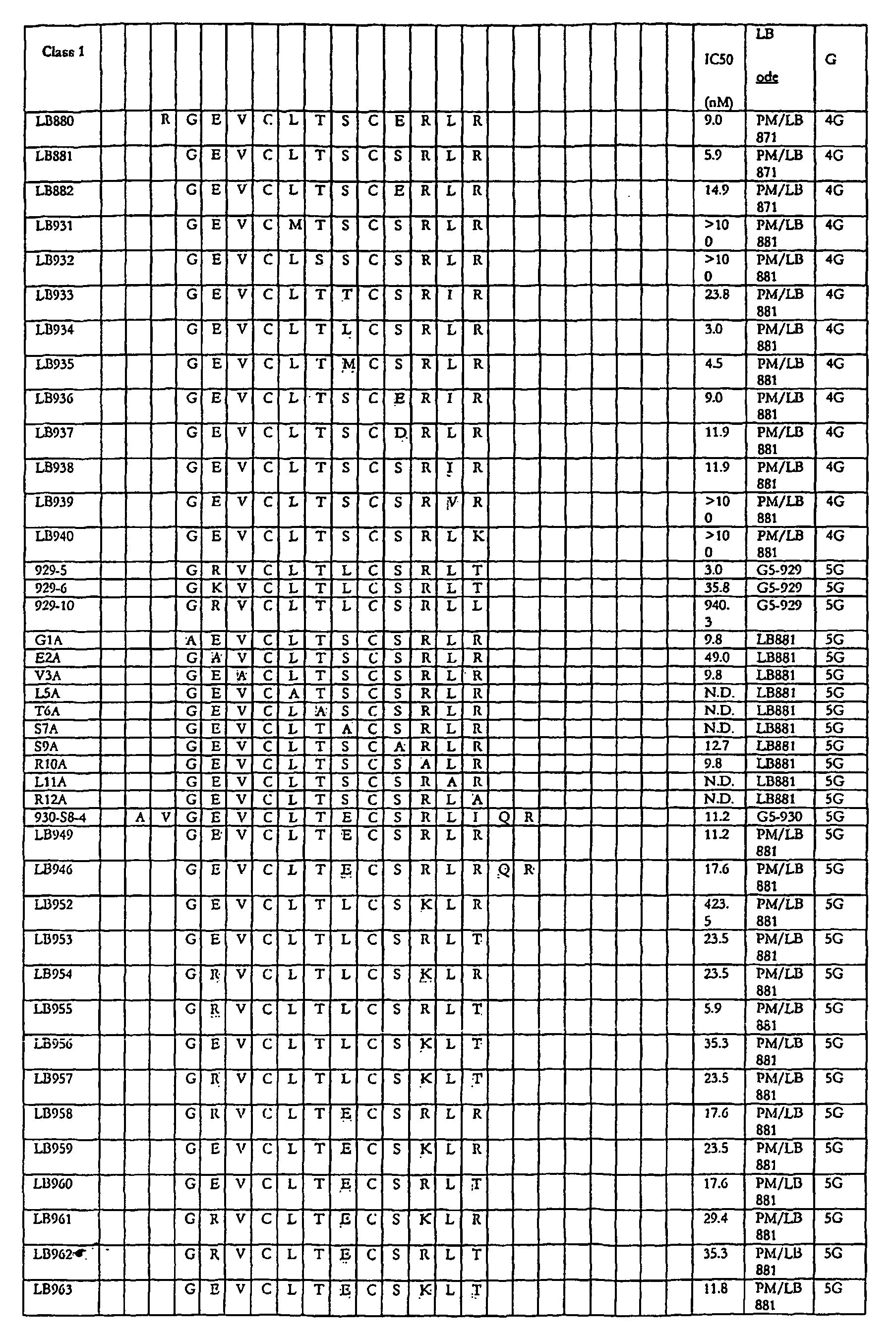

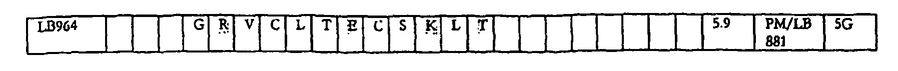

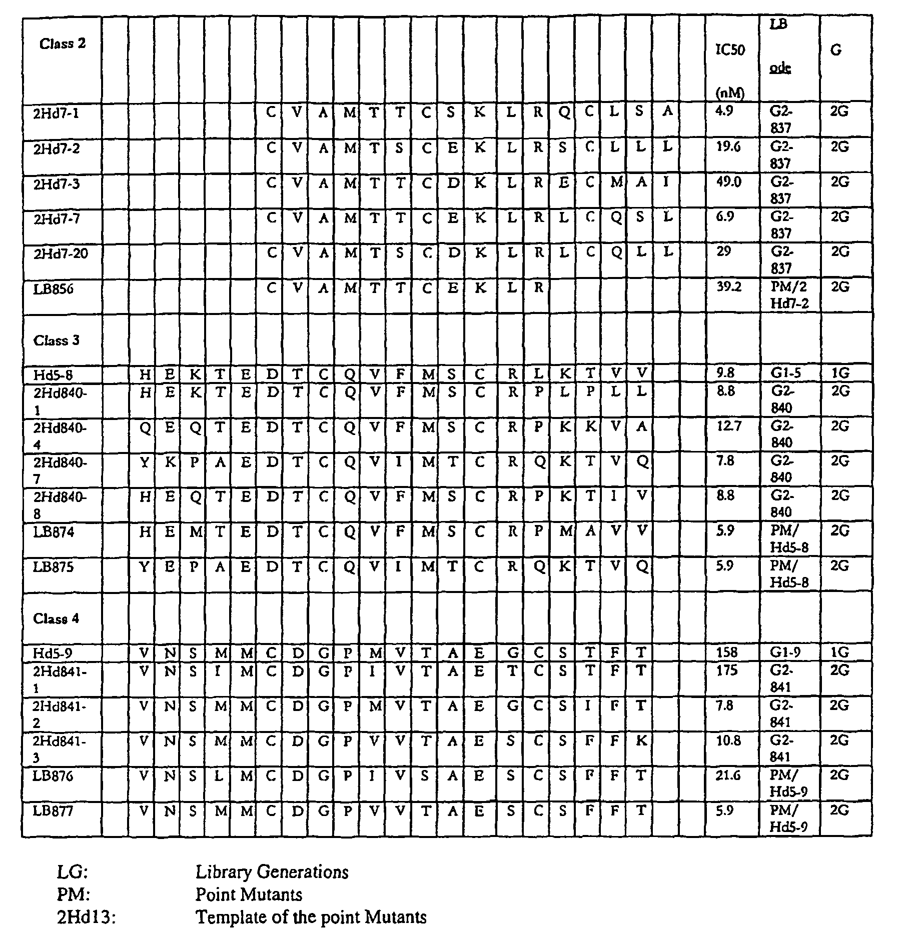

**TABLE IV: CERTAIN PROPERTIES OF SELECTED PEPTIDES:**

| CROSS-REACTIVITY | | | | | | | | | | |
|---|---|---|---|---|---|---|---|---|---|---|
| | | Mutants | Hu DR5 ECD (SEQID NO: 105) Ic50 nM | Hu DR5 IgG (SEQ ID NO: 109) Ic50 nM | Hu DR4 IgG (SEQ ID NO: 108) Ic50 nM | Cyno DR5 IgG (SEQ ID NO:112) Ic5O nM | Cyno DR4 IgG (SEQ ID NO:111) Ic50 nM | Hu DcR1 IgG (SEQ ID NO: 106) Ic50 nM | Hu DcR2 IgG (SEQ ID NO:107) Ic50 nM | Hu TNFR IgG (SEQ ID NO:110) Ic50 nM |
| 1 | C1 | Hd5-11 | 6.3 | 8 | 1000 | 10000 | 1000 | | | 1000 |
| 2 | C1 | 3H857-1 | 41.6 | 72 | 500 | 1000 | 200 | | | 300 |
| 3 | C1 | 3H858-4 | 66.7 | 10 | >5000 | >5000 | >5000 | | | >5000 |
| 4 | C1 | 3H856-5 | 50.0 | 20 | >5000 | >5000 | >5000 | | | >5000 |
| 5 | C1 | 870-22-S3 | 10.4 | 100 | 2000 | >5000 | 200 | | | >5000 |
| 6 | C1 | 870-23-S3 | 10.4 | 40 | >5000 | >5000 | 400 | | | >5000 |
| 7 | C1 | 870-2-S5 | 14.9 | 5 | >5000 | >5000 | >5000 | | | >5000 |
| 8 | C1 | 870-10-S5 | 11.9 | 50 | >5000 | >5000 | >5000 | | | >5000 |
| 9 | C1 | 871-44-S3* | 10.4 | 5 | 1000 | 79 | 56 | | | 5000 |
| 10 | C1 | LB880 | 9.0 | 1.3 | N.D. | N.D. | N.D. | | | N.D. |
| 11 | C1 | LB881* | 5.9 | 25 | N.D. | N.D. | N.D. | | | N.D. |
| 12 | C1 | LB949 | 112 | 25 | N.D. | N.D. | N.D. | | | N.D. |
| 13 | C1 | LB946 | 17.6 | 3.2 | N.D. | N.D. | N.D. | | | N.D. |
| 14 | C1 | LB952 | 423.5 | | N.D. | N.D. | N.D. | N.D. | N.D. | N.D. |
| 15 | C1 | LB953 | 23.5 | | N.D. | N.D. | N.D. | N.D. | N.D. | N.D. |
| 16 | C1 | LB954 | 23.5 | | >5000 | >5000 | >5000 | >5000 | >5000 | >5000 |
| 17 | C1 | LB955 * | 5.9 | 8.5 | >5000 | 4100 | >5000 | 2300 | 1300 | >5000 |
| 18 | C1 | LB956 | 35.3 | | N.D. | N.D. | N.D. | N.D. | N.D. | N.D. |
| 19 | C1 | LB957* | 23.5 | 8.6 | 2600 | 1100 | >5000 | >5000 | >5000 | 3200 |
| 20 | C1 | LB958 * | 17.6 | 9.6 | >5000 | >5000 | 2600 | 2000 | 2900 | >5000 |
| 21 | C1 | LB959 | 23.5 | | N.D. | N.D. | N.D. | N.D. | N.D. | N.D. |
| 22 | C1 | LB960 | 17.6 | | N.D. | N.D. | N.D. | N.D. | N.D. | N.D. |
| 23 | C1 | LB961 * | 29.4 | 3.0 | >5000 | >5000 | >5000 | 2600 | >5000 | >5000 |
| 24 | C1 | LB962 | 35.3 | 2.7 | 5000 | >5000 | 3200 | >5000 | 1000 | >5000 |
| 25 | C1 | LB963 | 11.8 | | N.D. | N.D. | N.D. | N.D. | N.D. | N.D. |
| 26 | C1 | LB964* | 5.9 | 6.6 | >5000 | >5000 | >5000 | >5000 | >5000 | >5000 |
| | | | | | | | | | | |
| 27 | C2 | LB856 * | 39.2 | 20 | N.D | N.D | N.D | | | N.D. |
| | | | | | | | | | | |
| 28 | C3 | LB874 | 5.9 | 25 | N.D. | N.D. | N.D. | | | N.D. |
| 29 | C3 | LB875 * | 5.9 | 5 | N.D. | N.D. | N.D. | | | N.D. |
| 30 | C3 | 2Hd840-1 | 8.8 | 13 | >5000 | | >5000 | | | >5000 |
| 31 | C3 | 2Hd840-4* | 12.7 | 10 | >5000 | | >5000 | | | 316 |
| | | | | | | | | | | |
| 32 | C4 | Hd5-9 | 158 | 6.3 | N.D. | 0.8 | N.D. | | | N.D. |
| 33 | C4 | 2Hd841-2 | 7.8 | 1.3 | >5000 | 0.3 | >5000 | | | 2000 |
| 34 | C4 | 2Hd841-3* | 10.8 | 0.3 | >5000 | 0.2 | >5000 | | | 125 |
| 35 | C4 | LB876 | 21.6 | 1.3 | N.D. | 0.4 | N.D. | | | N.D. |
| 36 | C4 | LB877 * | 5.9 | 0.8 | N.D. | 0.4 | N.D. | | | N.D. |

**TABLE V. GCN4 COILED COIL VARIANTS.**

| Positions of difference among the sequences are indicated in bold type. | | |
|---|---|---|
| oligomer | sequence | |
| dimer | KVKQLEDKVEELLSKNYHLENEVARLKKLVGER | (SEQ ID NO:8) |
| trimer | KIKQIEDKIEEILSKIYHIENEIARIKKLVGER | (SEQ ID NO:9) |
| tetramer | KLKQIEDKLEEILSKLYHIENELARIKKLVGER | (SEQ ID NO:10) |

**TABLE VI. LINKER VARIANTS.**

| linker code | sequence |
|---|---|
| L9 | GGSGGSGGM (SEQ ID NO:11) |
| L6 | GGSGGM (SEQ ID NO:12) |
| L4 | GGSM (SEQ ID NO:13) |
| L3 | GGM |
| L2 | GM |
| L1 | G |
| L0 | no linker |

**TABLE VII: IC 50 VALUES OF ILLUSTRATIVE PEPTIDES FUSED TO LEUCINE ZIPPER DOMAINS.**

| | **Ic 50 Elisa** | **Ec 50 bio-assay** |
|---|---|---|
| Peptide | | |
| 2Hd7-7-13A | | |
| 2Hd9-13 | | |
| | | |
| Peptide - L - Zipper (Linker: GGSGGM) | | |
| LB881-Monomer | | |
| LB881-Dimer | 125 nM | >37uM |
| LB881-Trimer | 14 nM | 37uM |
| LB881-Tetramer | 7nM | 166nM |
| | | |
| Peptide-LZ-Linker (LB881 Trimer) | | |
| LB881-noG-noM | | >37uM |
| LB881-G | | |
| LB881-GM | | 1688.7nM |
| LB881-GGM | | |
| LB881-GGSM | | 8450nM |
| LB881-GGSGGM | | 37uM |
| LB881-GGSGGSGGM | | |
| | | |

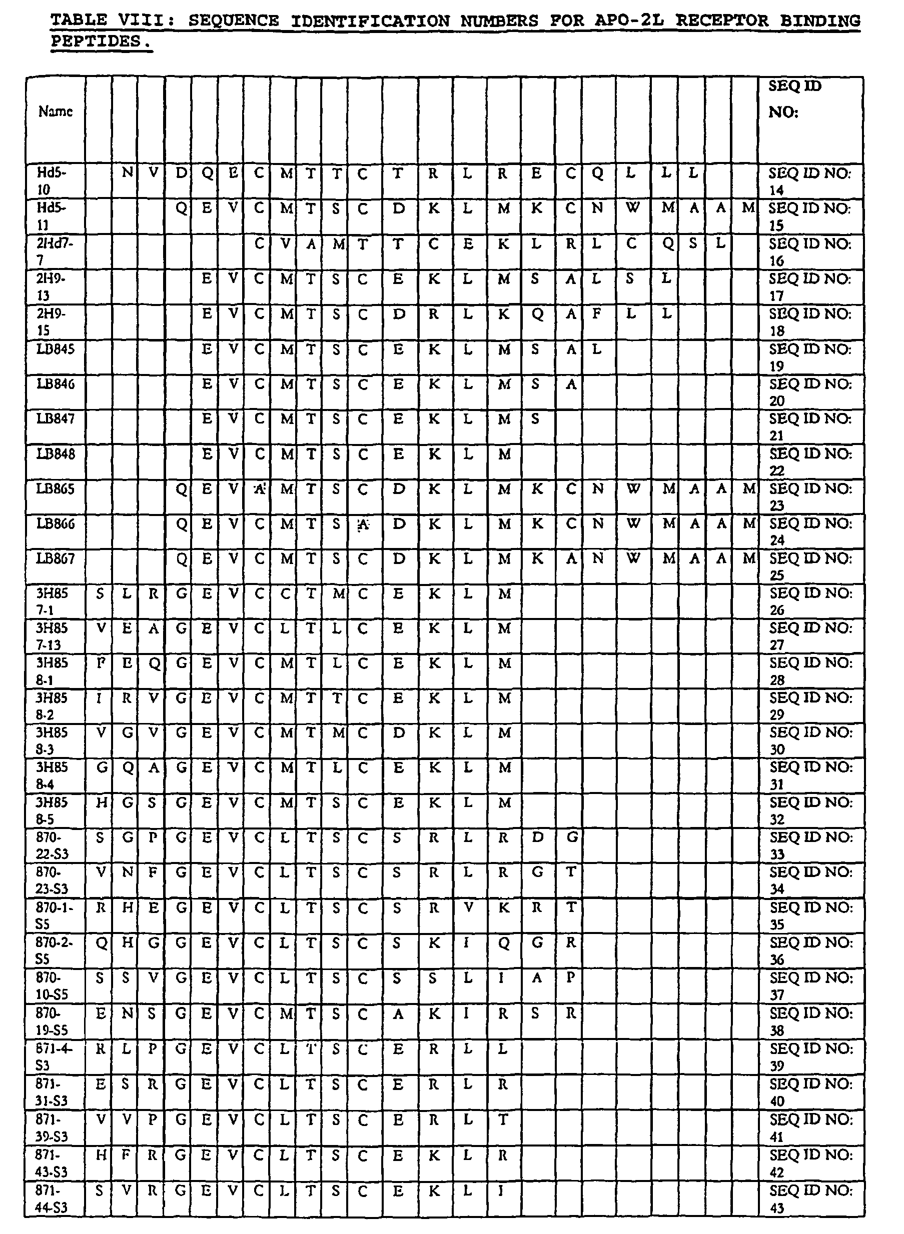

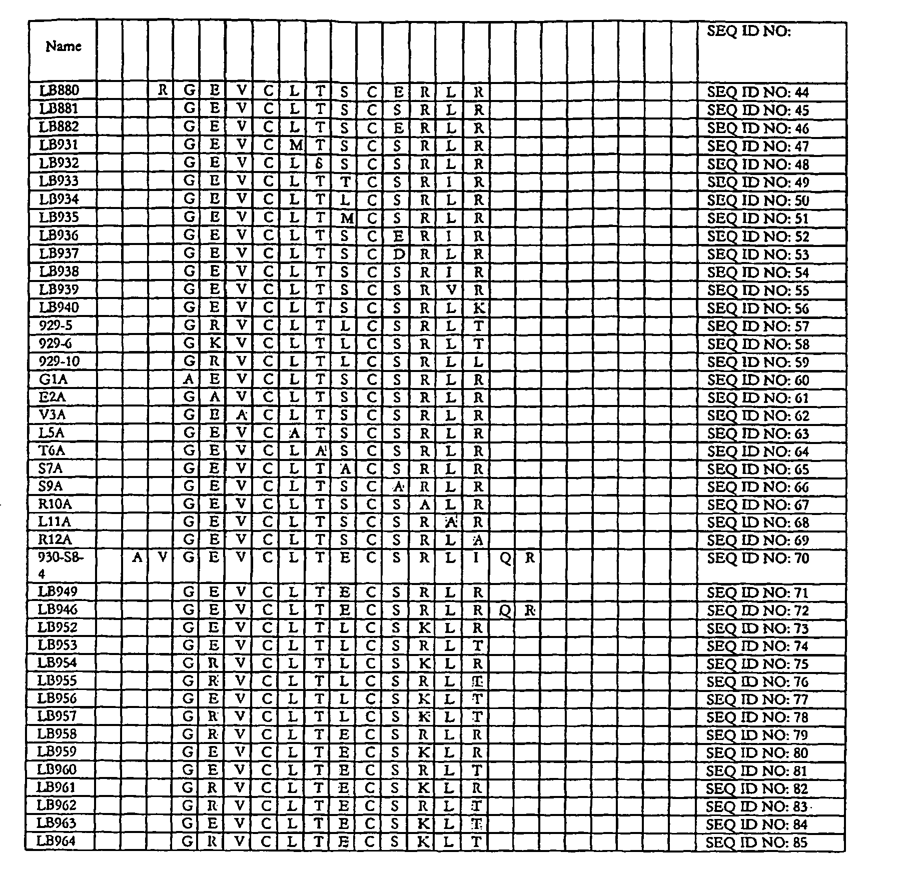

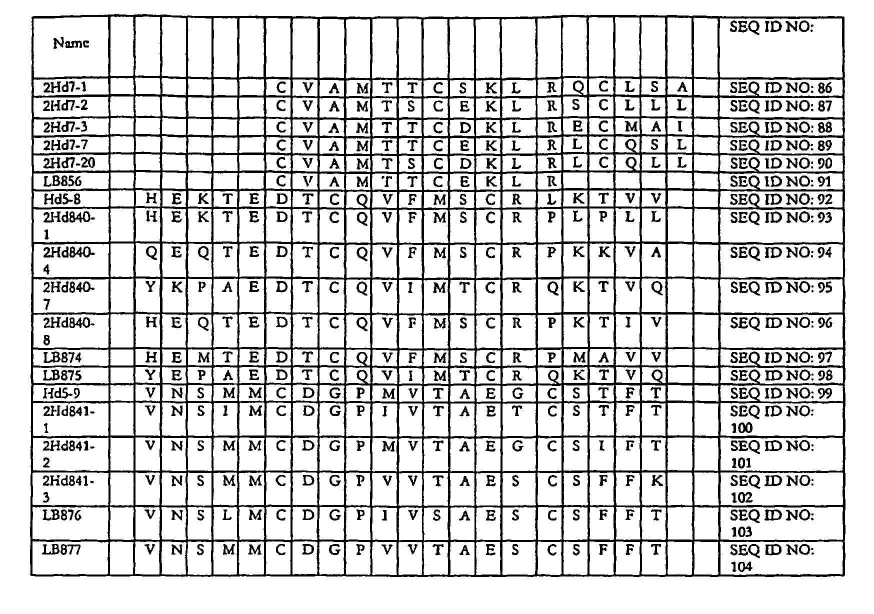

**TABLE IX: POLYPEPTIDE SEQUENCES OF REAGENTS USED TO CHARACTERIZE APO-2L RECEPTOR BINDING PEPTIDES.**

| 1. Human DRS-ECD long form polypeptide |
|---|
|  (SEQ ID NO: 105) |

| 2. Human DcR1 IgG fusion polypeptide |
|---|
| MARIPKTLKFVVVIVAVLLPVLAYSATTARQEEVPQQTVAPQQQRHSFKGEECPAGSHRS EHTGACNPCTEGVDYTNASNNEPSCFPCTVCKSDQKHKSSCTMTRDTVCQCKEGTFRNEN SPEMCRKCSRCPSGEVQVSNCTSWDDIQCVEEFGANATVETLAFQDKTHTCPPCPAPELL GGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSR EEMTKNQVSLTCLVKGFYPSDIAVEWESNGQPENNYKTTPPVLDSDGSFFLYSKLTVDKS RWQQGNVFSCSVMHEALHNHYTQKSLSLSPGK (SEQ ID NO: 106) |

| 3. Human DcR2 IgG fusion polypeptide |
|---|
|  (SEQ ID NO: 107) |

| 4. Human DR4 IgG fusion polypeptide |
|---|
| MAPPPARVHLGAFLAVTPNPGSAASGTEAAAATPSKVWGSSAGRIEPRGGGRGALPTSMG QHGPSARARAGRAPGPRPAREASPRLRVHKTFKFVVVGVLLQVVPSSAATIKLHDQSIGT QQWEHSPLGELCPPGSHRSERPGACNRCTEGVGYTNASNNLFACLPCTACKSDEEERSPC TTTRNTACQCKPGTFRNDNSAEMCRKCSTGCPRGMVKVKDCTPWSDIECVHKESGNGHND KTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKG QPREPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSPGK (SEQ ID NO: 108) |

| 5. Human DR5 IgG fusion polypeptide |
|---|
| MEQRGQNAPAASGARKRHGPGPREARGARPGLRVPKTLVLVVAAVLLLVSAESALITQQD LAPQQRAAPQQKRSSPSEGLCPPGHHISEDGRDCISCKYGQDYSTHWNDLLFCLRCTRCD SGEVELSPCTTTRNTVCQCEEGTFREEDSPEMCRKCRTGCPRGMVKVGDCTPWSDIECVH KESGLAFQDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALPAPI EKTISKAKGQPREPQVYTLPPSREEMTKNQVSLTCLVKGFYPSDIAVEWESNGQPENNYK TTPPVLDSDGSFFLYSKLTVDKSRWQQGNVFSCSVMHEALHNHYTQKSLSLSPGK (SEQ ID NO: 109) |

| 6. Human TNFR1 IgG fusion polypeptide |
|---|
| MGLSTVPDLLLPLVLLELLVGIYPSGVIGLVPHLGDREKRDSVCPQGKYIHPQNNSICCT KCHKGTYLYNDCPGPGQDTDCRECESGSFTASENHLRHCLSCSKCRKEMGQVEISSCTVD RDTVCGCRKNQYRHYWSENLFQCFNCSLCLNGTVHLSCQEKQNTVCTCHAGFFLRENECV SCSNCKKSLECTKLCLPQIENVKGTEDSGTTDKTHTCPPCPAPELLGGPSVFLFPPKPKD TLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPSREEMTKNQVSLTCLV KGFYPSDIAVEWESNGQPENNYKTTPPVLDSDGSFFLYSKLTVDKSRWQQGNVFSCSVMH EALHNHYTQKSLSLSPGK (SEQ ID NO: 110) |

| 7. Cyno DR4 IqG fusion polypeptide |
|---|
| MGQQGPSAQARAGRVVGPRSAQGASPGLRVHKTLKFVVVGVLLQVVPGSAATIKVHDQSV GTQQWEHSPLGELCPPGSHRSEHSGACNQCTEGVGYTSASNNLFSCLPCTACKSDEEERS |
| ACTRTRNTACQCKPGTFRNDDSAEMCRKCSTGCPRGKVKVKDCTPWSDIECVHNESGNGH NVWAILIVTVVILVVLLLLVAVLMFCRRIGSGCGGNPKCMHRVFLWCLGLLRGPGAEDNA HNMILNHGDSLSTFISEQQMESQEPADLTGVTVQSPGEAQCLLGPAEPEGSQRRRLLVPA NGADPTETMMLFFDNFADIVPFNSWDQLMRQLGLTNNEIHMVRADTAGPGDALYAMLMKW VNKTGQDASIHTLLDALERIGERHAKERIQDLLVDSGKFIYVEDGTGSAVSLE (SEQ ID NO: 111) |

| 8. Cyno DR5 IgG fusion polypeptide |
|---|
| MGQLRQSAPAASVARKGRGPGPREARGARPGLRVLKTLVLVVAAARVLLSVSADCAPITR QSLDPQRRAAPQQKRSSPTEGLCPPGHHISEDSRECISCKYGQDYSTHWNDFLFCLRCTK CDSGEVEVNSCTTTRNTVCQCEEGTFREEDSPEICRKCRTGCPRGMVKVKDCTPWSDIEC VHKESGIIIGVIVLVVIVVVTVIVWKTSLWKKVLPYLKGVCSGDGGDPEHVDSSSHSPQR PGAEDNALNEIVSIVQPSQVPEQEMEVQEPAEQTDVNTLSPGESEHLLEPAKAEGPQRRG QLVPVNENDPTETLRQCFDDFAAIVPFDAWEPLVRQLGLTNNEIKVAKAEAASSRDTLYV MLIKWVNKTGRAASVNTLLDALETLEERLAKQKIQDRLLSSGKFMYLEDNADSATS (SEQ ID NO: 112) |

## Claims

1. An isolated Apo-2L receptor binding peptide comprising an amino acid sequence selected from the group consisting of SEQ ID NO:14 through SEQ ID NO: 104 (Table VIII) for use in a method of treating cancer or an immune-related disease.

2. The Apo-2L receptor binding peptide for use in a method of treatment according to claim 1, wherein the cancer is lung cancer, breast cancer, colon cancer or colorectal cancer.

3. The Apo-2L receptor binding peptide for use in a method of treatment according to claim 1, wherein said immune-related disease is arthritis or multiple sclerosis.

4. The Apo-2L receptor binding peptide for use in a method of treatment according to any one of claims 1 to 3, wherein the peptide inhibits the interaction between a human DR4 receptor polypeptide and an Apo-2 ligand polypeptide comprising amino acids 114 to 281 of Figure 1.

5. The Apo-2L receptor binding peptide for use in a method of treatment according to any one of the preceding claims, wherein the peptide inhibits the interaction between a human DR5 receptor polypeptide and an Apo-2 ligand polypeptide comprising amino acids 114 to 281 of Figure 1.

6. The Apo-2L receptor binding peptide for use in a method of treatment according to any one of the preceding claims, wherein the peptide induces apoptosis in one or more mammalian cells.

7. The Apo-2L receptor binding peptide for use in a method of treatment according to any one of the preceding claims, wherein the peptide has a cysteine residue at position 1, 2, 3, 4, 5, 6, or 7 numbered from its N-terminus.

8. The Apo-2L receptor binding peptide for use in a method of treatment according to any one of the preceding claims, wherein the peptide includes the motif cysteine-X₁-X₂-X₃ -cysteine, wherein X₁ is an amino acid residue selected from the group consisting of methionine and leucine, X₂ is an amino acid residue selected from the group consisting of threonine and alanine residue, and X₃ is an amino acid residue selected from the group consisting of serine, methionine, leucine and glutamic acid.

9. The Apo-2L receptor binding peptide for use in a method of treatment according to any one of the preceding claims which binds a DR5 receptor but does not bind DR4 receptor.

10. The Apo-2L receptor binding peptide for use in a method of treatment according to any one of the preceding claims which binds a DR5 receptor and further binds to a DR4 receptor, a DcR1 receptor, and/or a DcR2 receptor.

11. The Apo-2L receptor binding peptide for use in a method of treatment according to any one of the preceding claims, wherein the Apo-2L receptor binding peptide is linked to a heterologous polypeptide sequence.

12. The Apo-2L receptor binding peptide for use in a method of treatment according to any one of the preceding claims, wherein the heterologous polypeptide sequence is a leucine zipper domain comprising the amino acid sequence glycine-glycine-methionine.

13. The Apo-2L receptor binding peptide for use in a method of treatment according to any one of the preceding claims, wherein the Apo-2L receptor binding peptide is conjugated or linked to one or more polyol groups.

14. The Apo-2L receptor binding peptide for use in a method of treatment according to any one of the preceding claims, wherein the peptide is formulated in a carrier.

15. The Apo-2L receptor binding peptide for use in a method of treatment according to claim 14, wherein the composition comprising the peptide is sterile.

16. The Apo-2L receptor binding peptide for use in a method of treatment according to any one of the preceding claims which is for administration with a further therapeutic agent.

## Patentansprüche

1. Isoliertes Apo-2L-Rezeptor-Bindungspeptid, umfassend eine Aminosäuresequenz, die aus der aus Seq.-ID Nr. 14 bis Seq.-ID Nr. 104 (Tabelle VIII) bestehenden Gruppe ausgewählt ist, zur Verwendung in einem Verfahren zur Behandlung von Krebs oder einer immunbezogenen Erkrankung.

2. Apo-2L-Rezeptor-Bindungspeptid zur Verwendung in einem Behandlungsverfahren nach Anspruch 1, worin der Krebs Lungenkrebs, Brustkrebs, Kolonkrebs oder kolorektaler Krebs ist.

3. Apo-2L-Rezeptor-Bindungspeptid zur Verwendung in einem Behandlungsverfahren nach Anspruch 1, worin die immunbezogene Krankheit Arthritis oder Multiple Sklerose ist.

4. Apo-2L-Rezeptor-Bindungspeptid zur Verwendung in einem Behandlungsverfahren nach einem der Ansprüche 1 bis 3, worin das Peptid die Wechselwirkung zwischen einem menschlichen DR4-Rezeptor-Polypeptid und einem Apo-2-Liganden-Polypeptid, das die Aminosäuren 114 bis 281 aus Fig. 1 umfasst, inhibiert.

5. Apo-2L-Rezeptor-Bindungspeptid zur Verwendung in einem Behandlungsverfahren nach einem der vorangegangenen Ansprüche, worin das Peptid die Wechselwirkung zwischen einem menschlichen DR5-Rezeptor-Polypeptid und einem Apo-2-Liganden-Polypeptid, das die Aminosäuren 114 bis 281 aus Fig. 1 umfasst, inhibiert.

6. Apo-2L-Rezeptor-Bindungspeptid zur Verwendung in einem Behandlungsverfahren nach einem der vorangegangenen Ansprüche, worin das Peptid Apoptose in einer oder mehreren Säugetierzellen induziert.

7. Apo-2L-Rezeptor-Bindungspeptid zur Verwendung in einem Behandlungsverfahren nach einem der vorangegangenen Ansprüche, worin das Peptid einen Cystein-Rest an den Positionen 1, 2, 3, 4, 5, 6 oder 7, nummeriert von seinem N-Terminus aus, aufweist.

8. Apo-2L-Rezeptor-Bindungspeptid zur Verwendung in einem Behandlungsverfahren nach einem der vorangegangenen Ansprüche, worin das Peptid das Motiv Cystein-X₁-X₂-X₃-Cystein umfasst, worin X₁ ein Aminosäurerest ist, der aus der aus Methionin und Leucin bestehenden Gruppe ausgewählt ist, X₂ ein Aminosäurerest ist, der aus der aus Threonin- und Alanin-Rest bestehenden Gruppe ausgewählt ist, und X₃ ein Aminosäurerest ist, der aus der aus Serin, Methionin, Leucin und Glutaminsäure bestehenden Gruppe ausgewählt ist.

9. Apo-2L-Rezeptor-Bindungspeptid zur Verwendung in einem Behandlungsverfahren nach einem der vorangegangenen Ansprüche, das einen DR5-Rezeptor, jedoch keinen DR4-Rezeptor bindet.

10. Apo-2L-Rezeptor-Bindungspeptid zur Verwendung in einem Behandlungsverfahren nach einem der vorangegangenen Ansprüche, das einen DR5-Rezeptor und weiters einen DR4-Rezeptor, einen DcR1-Rezeptor und/oder einen DcR2-Rezeptor bindet.

11. Apo-2L-Rezeptor-Bindungspeptid zur Verwendung in einem Behandlungsverfahren nach einem der vorangegangenen Ansprüche, worin das Apo-2L-Rezeptor-Bindungspeptid an eine heterologe Polypeptidsequenz gebunden ist.

12. Apo-2L-Rezeptor-Bindungspeptid zur Verwendung in einem Behandlungsverfahren nach einem der vorangegangenen Ansprüche, worin die heterologe Polypeptidsequenz eine Leucin-Zipper-Domäne ist, welche die Aminosäuresequenz Glycin-Glycin-Methionin umfasst.

13. Apo-2L-Rezeptor-Bindungspeptid zur Verwendung in einem Behandlungsverfahren nach einem der vorangegangenen Ansprüche, worin das Apo-2L-Rezeptor-Bindungspeptid an eine oder mehrere Polyol-Gruppen konjugiert oder gebunden ist.

14. Apo-2L-Rezeptor-Bindungspeptid zur Verwendung in einem Behandlungsverfahren nach einem der vorangegangenen Ansprüche, worin das Peptid in einem Träger formuliert ist.

15. Apo-2L-Rezeptor-Bindungspeptid zur Verwendung in einem Behandlungsverfahren nach Anspruch 14, worin die Zusammensetzung, die das Peptid umfasst, steril ist.

16. Apo-2L-Rezeptor-Bindungspeptid zur Verwendung in einem Behandlungsverfahren nach einem der vorangegangenen Ansprüche, das zur Verabreichung mit einem weiteren therapeutischen Mittel gedacht ist.

## Revendications

1. Peptide de liaison au récepteur d'Apo-2L isolé, comprenant une séquence d'amino-acides choisie dans le groupe consistant en les SEQ ID N° 14 à SEQ ID N° 104 (tableau VIII), destiné à être utilisé dans une méthode de traitement du cancer ou d'une maladie en rapport avec l'immunité.

2. Peptide de liaison au récepteur d'Apo-2L destiné à être utilisé dans une méthode de traitement suivant la revendication 1, le cancer étant le cancer du poumon, le cancer du sein, le cancer du côlon ou le cancer colorectal.

3. Peptide de liaison au récepteur d'Apo-2L destiné à être utilisé dans une méthode de traitement suivant la revendication 1, ladite maladie en rapport avec l'immunité étant l'arthrite ou la sclérose en plaques.

4. Peptide de liaison au récepteur d'Apo-2L destiné à être utilisé dans une méthode de traitement suivant l'une quelconque des revendications 1 à 3, ledit peptide inhibant l'interaction entre un polypeptide récepteur DR4 humain et un polypeptide ligand d'Apo-2 comprenant les amino-acides 114 à 281 de la figure 1.

5. Peptide de liaison au récepteur d'Apo-2L destiné à être utilisé dans une méthode de traitement suivant l'une quelconque des revendications précédentes, ledit peptide inhibant l'interaction entre un polypeptide récepteur DR5 humain et un polypeptide ligand d'Apo-2 comprenant les amino-acides 114 à 281 de la figure 1.

6. Peptide de liaison au récepteur d'Apo-2L destiné à être utilisé dans une méthode de traitement suivant l'une quelconque des revendications précédentes, ledit peptide induisant une apoptose dans une ou plusieurs cellules de mammifère .

7. Peptide de liaison au récepteur d'Apo-2L destiné à être utilisé dans une méthode de traitement suivant l'une quelconque des revendications précédentes, ledit peptide ayant un résidu cystéine en position 1, 2, 3, 4, 5, 6 ou 7 numérotée à partir de l'extrémité N-terminale de ce peptide.

8. Peptide de liaison au récepteur d'Apo-2L destiné à être utilisé dans une méthode de traitement suivant l'une quelconque des revendications précédentes, ledit peptide comprenant le motif cystéine-X₁-X₂-X₃-cystéine, dans lequel X₁ représente un résidu d'amino-acide choisi dans le groupe consistant en la méthionine et la leucine, X₂ représente un résidu d'amino-acide choisi dans le groupe consistant en la thréonine et l'alanine, et X₃ représente un résidu d'aminoacide choisi dans le groupe consistant en la sérine, la méthionine, la leucine et l'acide glutamique.

9. Peptide de liaison au récepteur d'Apo-2L destiné à être utilisé dans une méthode de traitement suivant l'une quelconque des revendications précédentes, qui se lie à un récepteur DR5 mais qui ne se lie pas à un récepteur DR4.

10. Peptide de liaison au récepteur d'Apo-2L destiné à être utilisé dans une méthode de traitement suivant l'une quelconque des revendications précédentes, qui se lie à un récepteur DR5 et qui se lie en outre à un récepteur DR4, à un récepteur DcR1 et/ou un récepteur DcR2.

11. Peptide de liaison au récepteur d'Apo-2L destiné à être utilisé dans une méthode de traitement suivant l'une quelconque des revendications précédentes, ledit peptide de liaison au récepteur d'Apo-2L étant lié à une séquence polypeptidique hétérologue.

12. Peptide de liaison au récepteur d'Apo-2L destiné à être utilisé dans une méthode de traitement suivant l'une quelconque des revendications précédentes, dans lequel la séquence polypeptidique hétérologue est un domaine de fermeture éclair leucine comprenant la séquence d'amino-acides glycine-glycine-méthionine.

13. Peptide de liaison au récepteur d'Apo-2L destiné à être utilisé dans une méthode de traitement suivant l'une quelconque des revendications précédentes, dans lequel le peptide de liaison au récepteur d'Apo-2L est conjugué ou lié à un ou plusieurs groupes polyol.

14. Peptide de liaison au récepteur d'Apo-2L destiné à être utilisé dans une méthode de traitement suivant l'une quelconque des revendications précédentes, ledit peptide étant formulé dans un support.

15. Peptide de liaison au récepteur d'Apo-2L destiné à être utilisé dans une méthode de traitement suivant la revendication 14, la composition comprenant le peptide étant stérile.

16. Peptide de liaison au récepteur d'Apo-2L destiné à être utilisé dans une méthode de traitement suivant l'une quelconque des revendications précédentes, qui est destiné à l'administration avec un agent thérapeutique supplémentaire.
